# FASCICULE DE BREVET EUROPEEN

(11) **EP 2 448 931 B1**
(45) Date de publication et mention de la délivrance du brevet: **27.05.2015**
(21) Numéro de dépôt: 10745347.4
(22) Date de dépôt: 28.06.2010
(51) Int. Cl.: C07D 401/12, C07D 295/15, A61K 31/40, A61K 31/4453, A61K 31/4709, A61K 31/5375, A61K 31/5377, A61P 35/00, A61P 25/00, A61P 29/00

(54) **Dérivés de benzène-sulfonamide, leur procédé de synthèse et leur utilisation en médecine**
Benzolsulfonamidverbindungen, Verfahren zu deren Herstellung und ihre Verwendung in der Medizin
Benzenesulfonamide compounds, method for synthesizing same, and use thereof in medicine

(30) Priorité: 30.06.2009 FR 0954459
(43) Date de publication de la demande: 09.05.2012
(73) Titulaire: Galderma Research & Development, 06410 Biot (FR)
(72) Inventeur: CLARY, Laurence, F-06480 La Colle sur Loup (FR); CHAMBON, Sandrine, F-06110 Le Cannet (FR); CHANTALAT, Laurent, 06600 Antibes (FR); MAUVAIS, Pascale, F-06600 Antibes (FR); ROYE, Olivier, F-83440 Fayence (FR); SCHUPPLI, Marlène, F-06650 Le Rouret (FR)
(74) Mandataire: Jansen, Cornelis Marinus
(86) Numéro de dépôt international: PCT/FR2010/051330
(87) Numéro de publication internationale: WO 2011/001088

(56) Documents cités:
- WO-A1-2008/045671
- US-A1- 2003 130 238
- US-B1- 6 326 516

## Description

### DOMAINE TECHNIQUE

La présente invention se rapporte à de nouveaux composés benzène-sulfonamides répondant à la formule (I) suivante : 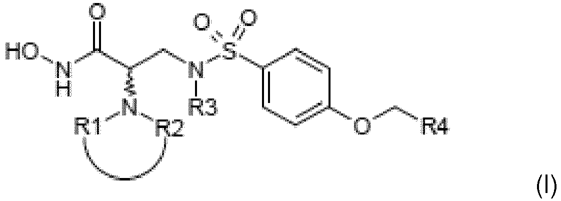 ainsi qu'à de tels composés comme un médicament et à leur utilisation dans des compositions pharmaceutiques destinées à un usage en médecine humaine ou vétérinaire.

Les composés de la présente invention agissent comme inhibiteurs de l'enzyme de conversion du TNFα encore appelée TACE. Ils sont de ce fait utiles dans le traitement des maladies pour lesquelles diminuer la production de TNFα est d'un grand intérêt.

### ETAT ANTERIEUR DE LA TECHNIG1UE

Les adamalysines (« ADAM » ou A Disintegrin and Metalloproteinase) sont une sous famille des enzymes métalloendopeptidases à zinc. Leur ectodomaine comporte un domaine protéasique dont l'activation dépend du zinc, un domaine disintégrine et un domaine riche en cystéines. A ce jour, au moins 30 ADAMs différentes ont été identifiées dont la première caractérisée a été ADAM17 encore appelée TACE (TNFα converting enzyme) [Gueydan C et al. Med.Sci 1997, 13, 83-88; Black R.A et al. Nature 1997, 385:729-733 ; Moss et al. Nature 1997, 385 :733-736]. L'ARNm de TACE est présent dans de nombreux tissus et plus particulièrement au niveau des monocytes, des macrophages, des lymphocytes T mais aussi au niveau des kératinocytes par exemple.

TACE est responsable de la coupure du pro-TNFα, protéine membranaire de 26 kDa, pour conduire à la libération du TNFα soluble, protéine de 17kDa, biologiquement active [Schlondorff et al. Biochem. J. 2000, 347, 131-138]. Le TNFα soluble libéré par la cellule est capable d'agir sur des sites très éloignés du site de synthèse.

Le TNFα est impliqué dans un grand nombre de processus biologiques pro-inflammatoires [Aggarwal et al. Eur. Cytokine Netw., 1996, 7: 93-124]. Plusieurs études pharmacologiques et cliniques ont montré de façon évidente que bloquer les effets du TNFα avec des anticorps spécifiques ou des biologiques anti-TNFα (Etanercept, Adalimumab, Infliximab) était bénéfique dans le traitement de maladies autoimmunes comme l'arthrite rhumatoide [Feldman et al. Lancet, 1994, 344, 1105), le diabète mélitus non insulino dépendant [Lohmander L.S et al. Arthritis Rheum, 1993, 36, 1214-1222], la maladie de Crohn's [MacDonald et al. Clin. Exp. Immunol. 1990, 81, 301]. US 6,326,516 B1 décrit des dérives de N-hydroxypropionamide utiles dans le traitement de maladies telles que l'arthrite.

Le TNFα joue également un rôle fondamental au cours du phénomène inflammatoire déclenché dans les lésions de psoriasis. Les taux sériques de TNFα sont élevés chez les patients psoriasiques [Mussi A et al. J. Biol. Regul. Homeost Agents, 1997, 11, 115-118] ; les taux de TNFα sont également élevés dans les plaques même de psoriasis [Bonifati C. et al. Clin. Exp. Dermatol., 1994, 19, 383-387]. Les cellules clés de la physiopathologie du psoriasis sont les kératinocytes, les cellules dendritiques et certains lymphocytes T. L'interaction entre ces familles de cellules conduit à une cascade inflammatoire menant aux lésions caractéristiques du psoriasis avec libération de TNFα [Kupper TS, N. Engl. J. Med, 2003, 349, 1987-1990]. Des études cliniques pour le traitement de psoriasis en plaques modéré à sévère par les anti-TNFα biologiques (Etanercept, Adalimumab, Infliximab) ont démontré leur efficacité à la fois sur les lésions psoriasiques et sur la qualité de vie des patients [Ortonne JP, Annales de dermatologie et de vénéreologie, 2005, 132 (8-9 pt2), 4S6-9 et 2005, 132, 9S01-9S70].

Ainsi, les composés qui inhibent la production de TNFα sont d'un grand intérêt pour le traitement des maladies inflammatoires et des maladies impliquant une libération de TNFα.

### EXPOSE DE L'INVENTION

La présente invention fournit des composés de benzène-sulfonamides répondant à la formule (I) comme définie en revendication 1. Les nouveaux composés inhibent l'enzyme TACE (TNFα converting enzyme) et de ce fait, inhibent la sécrétion de TNFα soluble (forme active du TNFα) par les cellules. Ces nouveaux composés sont donc des principes actifs potentiels pour le traitement des pathologies faisant intervenir une diminution ou une inhibition de la production de TNFα.

En conséquence, dans un autre aspect, la présente invention vise les composés répondant à la formule (I) pour leur utilisation dans le traitement des pathologies choisies dans le groupe consistant en le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'attaque ischémique, le rejet de greffe, le cancer, l'athérosclérose, l'obésité, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète melitus non insulino dépendant, les maladies allergiques, l'asthme, la maladie d'obstruction pulmonaire chronique (COPD), l'inflammation oculaire, les maladies inflammatoires de la peau, le psoriasis, la dermatite atopique et le rhumatisme psoriasique.

Ces composés sont également des principes actifs potentiels pour le traitement de pathologies neurologiques à caractère inflammatoire pour lesquelles diminuer la production de TNFα serait d'un grand intérêt. Dans encore un autre aspect de la présente invention les composés répondant à la formule (I) sont fournis pour leur utilisation dans le traitement des pathologies choisies dans le groupe consistant en la maladie d'Alzheimer, la maladie de Parkinson, les désordres parkinsoniens, la sclérose amyotrophique latérale, les maladies autoimmunes du système nerveux, les maladies autonomiques du système nerveux, les douleurs dorsales, l'oedème cérébral, les désordres cérébrovasculaires, la démence, les maladies autoimmunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barre, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux centreux, les maladies d'Huntington et l'attaque cérébrale ;

Une grande variété d'inhibiteurs de TACE est déjà connue (voir ci-dessous). Toutefois, un grand nombre de ces inhibiteurs n'agit pas sélectivement sur l'enzyme TACE par rapport à d'autres enzymes de la famille des ADAMs et/ou de matrix métalloprotéinases (MMPs).

Or, l'inhibition non sélective de ces familles d'enzymes induit des effets secondaires indésirables observés *In Vivo.* Par exemple, l'inhibition de MMP-1 (collagenase-1) a été associée aux problèmes de toxicité musculo-squelettique.

Comme inhibiteur non sélectif, on peut citer également l'Apratastat, inhibiteur connu et testé cliniquement en phase 2 pour le traitement de l'arthrite rhumatoïde (Curr Opin Investig Drugs. 2006 Nov;7(11):1014-9). Cet inhibiteur n'est pas sélectif de l'enzyme TACE par rapport à certaines MMPs (WO 00/44709 ; page 251 , table 10, exemple 61).

D'autres inhibiteurs de TACE également connus et faisant partie de la même famille que l'Apratastat, à savoir celle de dérivés benzène sulfonamides cycliques, ont été décrits dans WO 00/44709 et WO 97/18194. D'autres brevets (WO 96/00214, WO 97/22587) revendiquent des inhibiteurs de MMPs et/ou TACE pour lesquels la partie benzène sulfonamide est séparée de la fonction acide hydroxamique par un seul atome de carbone. Des publications décrivant ce type d'inhibiteurs de MMPs de façon plus large sont aussi celle de MacPherson et al. J. Med. Chem. 1997,40, 2525 et celle de Tamura et al. J. Med. Chem. 1998, 41, 640. D'autres exemples d'inhibiteurs de MMPs/TACE pour lesquels la fonction sulfonamide est séparée de l'acide hydroxamique par un enchainement de deux atomes de carbones formant un cycle sont décrits dans les brevets WO 98/16503, WO 98/16506, WO 98/16514 et WO 98/16520. D'autres exemples d'inhibiteurs de MMPs pour lesquels la fonction sulfonamide est séparée de l'acide hydroxamique par un enchainement de deux atomes de carbones sont également décrits dans WO 2008/045671.

Or, la demanderesse a maintenant découvert de manière inattendue et surprenante que de nouveaux composés de formule générale (I) présentent une très bonne activité inhibitrice de TACE et en particulier, inhibent l'enzyme TACE de façon sélective par rapport à d'autres ADAMs et MMPs.

Ainsi, la présente invention concerne des composés de formule générale (I) suivante : 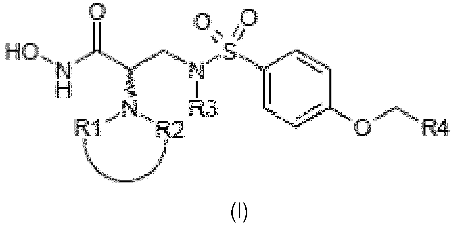 dans laquelle :
R₁ et R₂ forment un cycle avec l'atome d'azote auquel ils sont rattachés, ledit cycle étant représenté par la formule suivante : 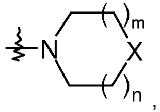
X, m et n ayant les significations données ci-après,
R₃ est un atome d'hydrogène ou un radical alkyle en C₁₋₄
R₄ est un radical alkyle en C₁₋₁₀, un radical alcényle en C₂₋₁₀, un radical alcynyle en C₂₋₁₀, un radical aryle, un radical alkyle en C₁₋₁₀ substitué par un radicale aryle non substitué ou substitué, un radical hétérocyclique, un radical cycloalkyle en C₃₋₇, un radical hétéroaryle, ou un radical hétéroaralkyle,;
X représente un atome d'oxygène, un radical -CH₂-, un atome de soufre, un radical SO ou un radical SO₂,
m peut prendre les valeurs de 0 ou 1 ; et
n peut prendre les valeurs de 0, 1, 2 ou 3 ;
ainsi que des sels d'addition dudit composé de formule (I) avec un acide pharmaceutiquement acceptable, des sels d'addition dudit composé de formule (I) avec une base pharmaceutiquement acceptable et des énantiomères dudit composé de formule (I), dans laquelle
lesdits radicaux alkyle en C₁₋₁₀, alcényle en C₂₋₁₀, alcynyle en C₂₋₁₀ et cycloalkyle en C₃₋₇ peuvent être substitués par un ou plusieurs radicaux choisis parmi un halogène, un alkoxyle en C₁₋₁₀ et un hydroxyle;
un radical aryle est un cycle hydrocarboné aromatique ou deux cycles condensés hydrocarbonés aromatiques;
un radical hétérocyclique est une chaîne hydrocarbonée cyclique ou polycyclique, saturée ou insaturée, comprenant un ou plusieurs hétéroatomes choisis parmi O, S et N;
lesquels radicaux aryle et hétérocyclique peuvent être substitués par un ou plusieurs groupes choisis parmi un alkyle en C₁₋₁₀, un alkoxyle en C₁₋₁₀, un aryle, un halogène, un hydroxyle, un cyano, un trifluorométhyle et un nitro;
un radical hétéroaryle est une chaîne hydrocarbonée cyclique ou polycyclique, aromatique, comprenant un ou plusieurs hétéroatomes choisis parmi O, S et N, lequel radical hétéroaryle peut être substitué par un ou plusieurs groupes choisis parmi un alkyle en C₁₋₁₀, un alkoxy en C₁₋₁₀, un aryle, un aryle substitué, un halogène, un hydroxy, un cyano, un trifluorométhyle et un nitro;
un radical hétéroaralkyle est un radical alkyle en C₁₋₁₀ substitué par un radical hétéroaryle, lequel radical hétéroaralkyle peut être substitué par un ou plusieurs groupes choisis parmi alkyle en C₁₋₁₀, alkoxyle en C₁₋₁₀, un halogène, un hydroxyle, un cyano, un trifluorométhyle et un nitro;
et
un halogène est un atome de fluor, de chlore, de brome ou d'iode.

Parmi les sels d'addition des composés de formule (I) avec un acide pharmaceutiquement acceptable, on peut citer de préférence les sels avec un acide organique ou avec un acide inorganique.

Les acides inorganiques appropriés sont par exemple les acides halohydriques comme l'acide chlorhydrique, l'acide bromhydrique, l'acide sulfurique, l'acide nitrique et l'acide phosphorique.

Les acides organiques appropriés sont par exemple l'acide acétique, l'acide trifluoroacétique, l'acide trichloroacétique, l'acide propionique, l'acide glycolique, l'acide pyruvique, l'acide succinique, l'acide benzoïque, l'acide cinnamique, l'acide mandélique, l'acide methanesulfonique, l'acide para-toluène sulfonique, l'acide salicylique, l'acide picrique, l'acide citrique, l'acide oxalique, l'acide tartrique, l'acide malonique, l'acide maléique, l'acide camphorsulfonique et l'acide fumarique.

Parmi les sels d'addition des composés de formule (I) avec une base pharmaceutiquement acceptable, on peut citer de préférence les sels avec une base organique ou avec une base inorganique.

Les bases inorganiques appropriées sont les hydroxydes de métaux alcalins ou de métaux alcalino-terreux. Parmi ces bases, on peut citer par exemple l'hydroxyde de potassium, l'hydroxyde de sodium, l'hydroxyde de lithium ou encore l'hydroxyde de calcium.

Les bases organiques appropriés comprennent les amines et les amino-acides. Parmi les amines, on peut citer par exemple les amines primaires, secondaires ou tertiaires aliphatiques ou aromatiques comme la méthylamine, l'éthylamine, l'éthanolamine, la propylamine, l'isopropylamine, les 4 isomères de la butylamine, la diméthylamine, la diéthylamine, la diéthanolamine, la dipropylamine, la diisopropylamine, la di n-butylamine, la pyrrolidine, la pipéridine, la morpholine, la diéthanol phénylamine, la triméthylamine, la triéthylamine, la tripropylamine, la quinuclidine, la pyridine, la quinoline ou l'isoquinoline. Parmi les amino-acides, on peut citer par exemple la lysine, l'arginine et l'ornithine.

On désigne par radical alkyle inférieur une chaîne hydrocarbonée saturée, linéaire ou ramifiée, comprenant de 1 à 4 atomes de carbone.

On désigne par radical alkyle une chaîne hydrocarbonée saturée, linéaire ou ramifiée, comprenant de 1 à 10 atomes de carbone.

On désigne par radical alcényle une chaîne hydrocarbonée insaturée, linéaire ou ramifiée comprenant de 2 à 10 atomes de carbone et comprenant une ou plusieurs doubles liaisons.

On désigne par radical alcynyle une chaîne hydrocarbonée insaturée, linéaire ou ramifiée comprenant de 2 à 10 atomes de carbone et comprenant une ou plusieurs triples liaisons.

On désigne par radical alkyle substitué une chaîne hydrocarbonée saturée, linéaire ou ramifiée, comprenant de 1 à 10 atomes de carbone et substitué par un ou plusieurs radicaux choisis parmi un atome d'halogène, un radical alkoxyle et un radical hydroxyle.

On désigne par radical alcényle substitué une chaîne hydrocarbonée insaturée, linéaire ou ramifiée, comprenant de 2 à 10 atomes de carbone, comprenant une ou plusieurs doubles liaisons et substituée par un ou plusieurs radicaux choisis parmi un atome d'halogène, un radical alkoxyle et un radical hydroxyle.

On désigne par radical alcynyle substitué une chaîne hydrocarbonée insaturée, linéaire ou ramifiée, comprenant de 2 à 10 atomes de carbone, comprenant une ou plusieurs triples liaisons et substituée par un ou plusieurs radicaux choisis parmi un atome d'halogène, un radical alkoxyle et un radical hydroxyle.

On désigne par cycloalkyle une chaine hydrocarbonée, saturée, cyclique comprenant de 3 à 7 atomes de carbones.

On désigne par cycloalkyle substitué une chaine hydrocarbonée, saturée, cyclique comprenant de 3 à 7 atomes de carbones et substitué par un ou plusieurs radicaux choisis parmi un atome d'halogène, un radical alkoxyle et un radical hydroxyle.

On désigne par radical aryle, un cycle hydrocarboné aromatique ou deux cycles fusionnés hydrocarbonés aromatiques.
Les radicaux aryles préférés sont choisis parmi les radicaux phenyle et naphtyle.

On désigne par radical aryle substitué, un cycle ou deux cycles fusionnés hydrocarboné(s) aromatique(s) substitué(s) par un ou plusieurs groupes d'atomes choisis parmi un alkyle, un alkoxyle, un aryle, un halogène, un hydroxyle, un cyano, un trifluorométhyle et un nitro.

On désigne par radical aralkyle, un alkyle substitué par un aryle.

On désigne par radical aralkyle substitué, un alkyle substitué par un aryle substitué.
On désigne par radical hétérocyclique une chaîne hydrocarbonée cyclique ou polycyclique, saturée ou insaturée, comprenant un ou plusieurs hétéroatomes choisis parmi O, S et N.

On désigne par radical hétérocyclique substitué, un radical hétérocyclique substitué par un ou plusieurs groupes d'atomes choisis parmi un alkyle, un alkoxyle, un halogène, un hydroxyle, un cyano, un trifluorométhyle et un nitro.

On désigne par radical hétéroaryle, un radical hétérocyclique aromatique c'est-à-dire une chaîne hydrocarbonée cyclique ou polycyclique, aromatique, comprenant un ou plusieurs hétéroatomes choisis parmi O, S et N.

On désigne par radical hétéroaryle substitué, un radical hétéroaryle substitué par un ou plusieurs groupes d'atomes choisis parmi un alkyle, un alkoxy, un aryle, un aryle substitué, un halogène, un hydroxy, un cyano, un trifluorométhyle et un nitro.

On désigne par radical hétéroaralkyle, un radical alkyle substitué par un radical hétéroaryle.

On désigne par radical hétéroaralkyle substitué, un radical hétéroaralkyle substitué par un ou plusieurs groupes d'atomes choisis parmi un alkyle, un alkoxyle, un halogène, un hydroxyle, un cyano, un trifluorométhyle et un nitro.

On désigne par radical alkoxyle un atome d'oxygène substitué par un radical alkyle.

On désigne par atome d'halogène un atome de fluor, de chlore, de brome ou d'iode.

Parmi les composés de formule (I) entrant dans le cadre de la présente invention, on peut notamment citer les composés suivants :
1) (S)-3-(4-benzyloxy-benzènesulfonylamino)-N-hydroxy-2-pipéridin-1-yl-propionamide
2) (S)-3-(4-benzyloxy-benzènesulfonylamino)-N-hydroxy-2-pyrrolidin-1-yl-propionamide
3) (S)-3-[4-(4-fluoro-benzyloxy)-benzènesulfonylamino]-N-hydroxy-2-pipéridin-1-yl-propionamide
4) (S)-3-[(4-benzyloxy-benzènesulfonyl)-méthyl-amino]-N-hydroxy-2-pipéridin-1-yl-propionamide
5) (S)-N-hydroxy-3-[4-(naphthalen-2-ylméthoxy)-benzènesulfonylamino]-2-pipéridin-1-yl-propionamide
6) (S)-3-[4-(3,4-dichloro-benzyloxy)-benzènesulfonylamino]-N-hydroxy-2-pipéridin-1-yl-propionamide
7) (S)-3-(4-benzyloxy-benzènesulfonylamino)-N-hydroxy-2-morpholin-4-yl-propionamide
8) (S)-N-hydroxy-3-[4-(2-méthyl-quinolin-4-ylméthoxy)-benzènesulfonylamino]-2-pipéridin-1-yl-propionamide
9) (S)-3-[4-(3,5-dichloro-benzyloxy)-benzènesulfonylamino]-N-hydroxy-2-pipéridin-1-yl-propionamide
10) (S)-N-hydroxy-2-pipéridin-1-yl-3-(4-propoxy-benzènesulfonylamino)-propionamide
11) (S)-3-(4-cyclopropylméthoxy-benzènesulfonylamino)-N-hydroxy-2-morpholin-4-yl-propionamide
12) (S)-3-[4-(4-tert-butyl-benzyloxy)-benzènesulfonylamino]-N-hydroxy-2-morpholin-4-yl-propionamide
13) (S)-N-hydroxy-2-morpholin-4-yl-3-(4-phénéthyloxy-benzènesulfonylamino)-propionamide
14) (S)-N-hydroxy-3-[4-(2-méthyl-quinolin-4-ylméthoxy)-benzènesulfonylamino]-2-morpholin-4-yl-propionamide
15) Di chlorhydrate de (S)-N-hydroxy-3-[4-(2-méthyl-quinolin-4-ylméthoxy)-benzènesulfonylam ino]-2-pipéridin-1-yl-propionam ide
16) (R)-N-hydroxy-3-[4-(2-méthyl-quinolin-4-ylméthoxy)-benzènesulfonylamino]-2-pyrrolidin-1-yl-propionamide
17) (R)-N-hydroxy-3-[4-(2-méthyl-quinolin-4-ylméthoxy)-benzènesulfonylamino]-2-pipéridin-1-yl-propionamide
18) (S)-N-hydroxy-3-[4-(2-méthyl-quinolin-4-ylméthoxy)-benzènesulfonylamino]-2-thiomorpholin-4-yl-propionamide
19) (S)-2-(1,1-dioxo-thiomorpholin-4-yl)-N-hydroxy-3-[4-(2-méthyl-quinolin-4-ylméthoxy)-benzènesulfonylamino]-propionamide
20) (S)-2-azepan-1-yl-N-hydroxy-3-[4-(3-méthyl-benzyloxy)-benzènesulfonylamino]-propionamide
21) (S)-2-azepan-1-yl-N-hydroxy-3-[4-(2-méthyl-quinolin-4-ylméthoxy)-benzènesulfonylamino]-propionamide
22) (S)-N-hydroxy-2-pipéridin-1-yl-3-[4-(pyrazolo[1,5-a]pyridin-3-ylméthoxy)-benzènesulfonylamino]-propionamide
23) (S)-N-hydroxy-3-[4-(2-méthyl-pyrazolo[1,5-a]pyridin-3-ylméthoxy)-benzènesulfonylamino]-2-pipéridin-1-yl-propionam ide
24) (S)-N-hydroxy-2-pipéridin-1-yl-3-[4-(quinolin-4-ylméthoxy)-benzènesulfonylamino]-propionamide
25) (S)-N-hydroxy-2-morpholin-4-yl-3-[4-(quinolin-4-ylméthoxy)-benzènesulfonylamino]-propionamide
26) (S)-N-hydroxy-3-{propyl-[4-(quinolin-4-ylméthoxy)-benzènesulfonyl]-amino}-2-pyrrolidin-1-yl-propionamide
27) (S)-N-hydroxy-3-[4-(3-méthyl-benzyloxy)-benzènesulfonylamino]-2-[1,4]-oxazocan-4-yl-propionamide
28) (S)-2-azocan-1-yl-N-hydroxy-3-[4-(pyrimidin-4-ylméthoxy)-benzènesulfonylamino]-propionamide
29) (S)-N-hydroxy-2-morpholin-4-yl-3-[4-(pyrazolo[1,5-a]pyridin-3-ylméthoxy)-benzènesulfonylamino]-propionamide
30) (S)-N-hydroxy-3-[4-(pyrazolo[1,5-a]pyridin-3-ylméthoxy)-benzènesulfonylamino]-2-thiomorpholin-4-yl-propionamide
31) (S)-N-hydroxy-2-pipéridin-1-yl-3-[4-(4-propoxy-benzyloxy)-benzènesulfonylamino]-propionamide
32) (R)-N-hydroxy-3-[4-(2-méthyl-quinolin-4-ylméthoxy)-benzènesulfonylamino]-2-morpholin-4-yl-propionamide.
33)(S)-N-hydroxy-3-[4-(2-méthyl-1 H-indol-3-ylméthoxy)-benzènesulfonylamino]-2-pipéridin-1-yl-propionamide
34)(S)-N-hydroxy-3-[4-(2-isopropyl-benzofuran-3-ylméthoxy)-benzènesulfonylamino]-2-morpholin-4-yl-propionamide
35)(S)-2-azétidin-1-yl-N-hydroxy-3-[4-(2-méthyl-quinolin-4-ylméthoxy)-benzènesulfonylamino]-propionamide

Les composés de formule **(I)** sont préparés selon le schéma réactionnel de la figure 1 présenté ci-dessous. 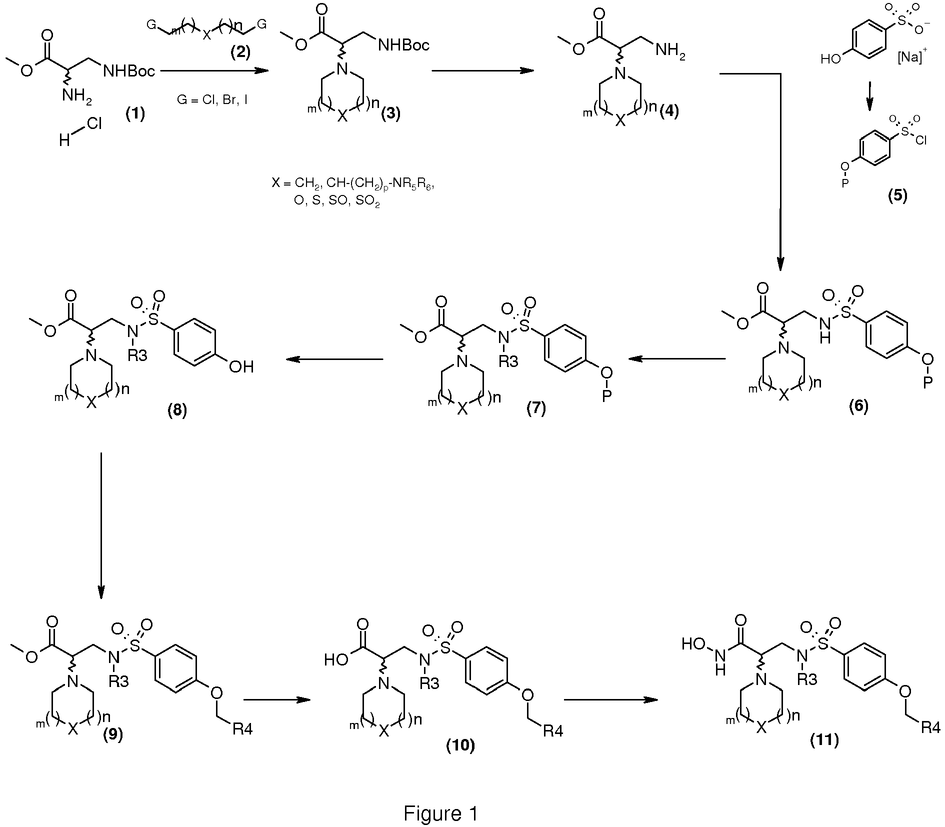 X = CH₂, O, S, SO, SO₂

Selon la figure 1, les composés **(3)** sont obtenus par réaction entre l'acide aminé **(1)** H-DAP(Boc)-OMe.HClou H-(D)-DAP(Boc)-OMe.HCl et le composé **(2)** (commercial ou préalablement préparé) en présence d'une base tertiaire organique comme la diisopropyléthylamine ou la triéthylamine à une température comprise entre 60°C et 120°C. Les composés **(4)** sont obtenus par déprotection de la fonction amine des composés **(3)** selon des méthodes classiques comme par exemple l'utilisation d'une solution d'acide chlorhydrique dans l'isopropanol.

Une réaction entre le composé **(4)** et le chlorure de 4-hydroxy-benzène sulfonyle O-protégé par un groupement benzyle **(5)** en présence d'une base tertiaire comme par exemple la triéthylamine dans du dichlorométhane conduit au composé **(6)**. Une N-alkylation de la fonction sulfonamide peut alors être réalisée par réaction avec un halogénure d'alkyle en présence d'une base comme par exemple du carbonate de potassium dans un solvant tel que du DMF pour conduire au dérivé **(7)**. Le composé **(8)** est obtenu par déprotection selon les méthodes connues par l'homme de l'art pour déprotéger une fonction phénol. Le composé **(9)** est obtenu par alkylation de la fonction phénol du composé **(8)** par réaction avec un halogénure d'alkyle en présence d'une base comme par exemple du carbonate de césium dans de l'acétone ou par réaction de Mitsunobu avec un dérivé alcool primaire en présence de triphénylphosphine et de diisopropyl azodicarboxylate par exemple. Par réaction de saponification en présence d'une base comme l'hydroxyde de lithium en présence d'eau et de tétrahydrofuranne par exemple, le composé **(10)** est obtenu. Dans une dernière étape, le composé **(11)** est obtenu par couplage entre la O-(tert-butyldimethylsilyl)hydroxylamine par exemple et le dérivé **(10)** dans des conditions de couplage peptidique classiques, en utilisant par exemple comme agents de couplage le chlorhydrate de 1-(3-diméthylaminopropyl)-3-éthylcarbodiimide, l'hydroxybenzotriazole ou le TBTU, et comme base la triéthylamine ou la diisopropyléthylamine dans un solvant tel que le dichlorométhane ou le diméthylformamide. La déprotection de l'acide hydroxamique silylé intermédiairement formé se fait in situ ou par lavage avec une solution aqueuse acide pour conduire au composé **(11)**.

Une autre alternative pour l'obtention du composé **(11)** est présentée dans la figure 2 ci-dessous.

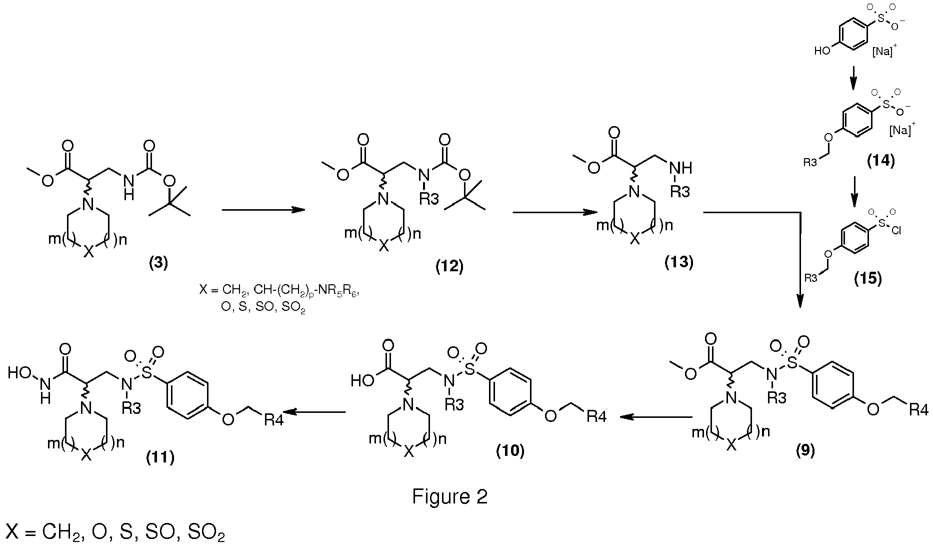

Selon le schéma de synthèse de la figure 2, le dérivé **(3)** peut éventuellement être alkylé en présence d'une base telle que l'hydrure de sodium et d'un halogénure d'alkyle dans du diméthylformamide par exemple pour conduire au composé **(12)** à partir duquel le composé (13) est obtenu selon les méthodes classiques de déprotection des amines comme par exemple l'utilisation d'une solution d'acide chlorhydrique dans l'isopropanol.

Le composé **(14)** est préalablement préparé à partir du sel de sodium de l'acide 4-hydroxy benzènesulfonique commercial par alkylation avec un halogénure d'alkyle en présence d'une base telle que l'hydroxyde de sodium par exemple dans un mélange de solvants comme l'isopropanol et l'eau par exemple. Le composé **(15)** est alors obtenu par réaction du dérivé **(14)** avec du chlorure d'oxalyle en présence de diméthylformamide dans du dichlorométhane par exemple.
Le dérivé **(9)** est obtenu par réaction entre les composés **(13)** et **(15)** en présence d'une base comme la triéthylamine dans du dichlorométhane par exemple.

Les composés de formule (I) préférés sont ceux pour lesquels :
R₄ est un radical aryle, un radical alkyle en C₁₋₁₀, un radical alkyle en C₁₋₁₀ substitué par un radicale aryle non substitué ou substitué, un radical hétérocyclique, un radical hétéroaryle, ou
un radical hétéroaralkyle; et
n peut prendre les valeurs de 0, 1 ou 2;
ainsi que des sels d'addition dudit composé avec un acide pharmaceutiquement acceptable, des sels d'addition dudit composé avec une base pharmaceutiquement acceptable et des énantiomères dudit composé.

Les composés de formule (I) particulièrement préférés sont ceux pour lesquels :
R₄ est un radical aryle, un radical alkyle en C₁₋₁₀, un radical alkyle en C₁₋₁₀ substitué par un radicale aryle non substitué ou substitué, un radical hétérocyclique, un radical hétéroaryle, ou
un radical hétéroaralkyle,;
X représente un atome d'oxygène, ou un radical -CH₂-,
m prend la valeur 1 ; et
n peut prendre les valeurs de 0, 1 ou 2;
ainsi que des sels d'addition dudit composé avec un acide pharmaceutiquement acceptable, des sels d'addition dudit composé avec une base pharmaceutiquement acceptable et des énantiomères dudit composé.

Les composés de formule (I) plus particulièrement préférés sont ceux pour lesquels :
R₃ est un atome d'hydrogène,
X représente un atome d'oxygène, ou un radical -CH₂- ,
m prend la valeur 1 ; et
n peut prendre les valeurs de 1 ou 2;
ainsi que des sels d'addition dudit composé avec un acide pharmaceutiquement acceptable, des sels d'addition dudit composé avec une base pharmaceutiquement acceptable et des énantiomères dudit composé.

Les composés de formule (I) encore plus particulièrement préférés sont ceux pour lesquels :
R₃ est un atome d'hydrogène,
R₄ est un radical hétérocyclique, un radical hétéroaryle, ou un radical hétéroaralkyle;
X représente un atome d'oxygène ou un radical -CH₂-;
m prend la valeur 1 ; et
n prend la valeur 1;
ainsi que des sels d'addition dudit composé avec un acide pharmaceutiquement acceptable, des sels d'addition dudit composé avec une base pharmaceutiquement acceptable et des énantiomères dudit composé.

Les composés de formule (I) les plus particulièrement préférés sont ceux pour lesquels :
R₃ est un atome d'hydrogène,
R₄ est un radical hétéroaryle,
X représente un atome d'oxygène ou un radical -CH₂-,
m prend la valeur 1 ; et
n prend la valeur 1;
ainsi que des sels d'addition dudit composé avec un acide pharmaceutiquement acceptable, des sels d'addition dudit composé avec une base pharmaceutiquement acceptable et des énantiomères dudit composé.

Les composés selon l'invention présentent une très bonne activité inhibitrice de TACE et en particulier, inhibent l'enzyme TACE de façon sélective par rapport à d'autres ADAMs et MMPs. Cette activité inhibitrice de l'enzyme TACE est mesurée dans un test enzymatique et quantifiée par la mesure d'une IC₅₀ (concentration d'inhibiteur nécessaire pour obtenir 50% d'inhibition de l'enzyme TACE), comme décrit dans l'exemple 17. Les composés de la présente invention présentent une IC₅₀ pour TACE inférieure ou égale à 10µM et plus particulièrement inférieure ou égale à 1µM. De façon avantageuse, les composés de la présente invention présentent une IC₅₀ pour TACE inférieure ou égale à 0,5µM.

De façon avantageuse, ces composés sont également très sélectifs de TACE par rapport aux autres ADAMs et MMPs (voir exemple 18) : leur activité inhibitrice est au moins 10 fois plus importante pour TACE que pour d'autres ADAMs et MMPs (c'est-à-dire que la valeur de l'IC₅₀ pour TACE est au moins 10 fois plus petite que celle pour d'autres ADAMs et MMPs), et plus avantageusement au moins 100 fois plus importante.

TACE (TNFα-Converting Enzyme) catalyse la formation du TNF-alpha soluble à partir de la protéine précurseur (TNFα transmembranaire) liée aux membranes de certaines cellules. Le TNFα est une cytokine pro-inflammatoire qui est connue pour jouer un rôle dans de nombreuses pathologies à caractère inflammatoire.

L'invention vise donc aussi un composé de formule (I) tel que défini ci-dessus pour son utilisation dans le traitement de pathologies ou de désordres liés à une libération de TNFα comme définie en revendications 11 - 13. Un inhibiteur de l'enzyme TACE de formule générale (I) diminue la production de TNFα. De ce fait, il est utile pour le traitement de pathologies liées à une libération et production de TNFα.

L'invention vise aussi une composition pharmaceutique comprenant d'au moins un composé de formule (I) tel que défini ci-dessus dans un support pharmaceutiquement acceptable

La méthode de traitement thérapeutique (humain ou animal), consiste en l'administration ou l'application d'une composition pharmaceutique comprenant un composé de formule (I) en tant qu'inhibiteur de TACE et de ce fait en tant qu'inhibiteur de la production de TNFα soluble.

L'invention concerne également l'utilisation d'un composé de formule (I) tel que défini ci-dessus pour la préparation d'un médicament destiné au traitement de pathologies pour lesquelles diminuer la production de TNFα serait d'un grand intérêt.

En effet, les composés selon l'invention sont particulièrement appropriés au traitement des désordres / maladies tels que les maladies inflammatoires listées ci-après: le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'athérosclérose, l'obésité, l'attaque ischémique, le rejet de greffe, le cancer, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète melitus non insulino dépendant, les maladies allergiques, l'asthme, la maladie d'obstruction pulmonaire chronique (COPD), les maladies inflammatoires de la peau, le psoriasis, la dermatite atopique et le rhumatisme psoriasique.

Ces composés sont également des principes actifs potentiels pour le traitement de pathologies neurologiques à caractère inflammatoire pour lesquelles diminuer la production de TNFα serait d'un grand intérêt. Ces pathologies listées ci-après sont par exemple la maladie d'Alzheimer, la maladie de Parkinson, les désordres parkinsoniens, la sclérose amyotrophique latérale, les maladies autoimmunes du système nerveux, les maladies autonomiques du système nerveux, les douleurs dorsales, l'oedème cérébral, les désordres cérébrovasculaires, la démence, les maladies autoimmunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barre, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux centreux, les maladies d'Huntington et l'attaque cérébrale ;

L'invention concerne l'utilisation d'un composé de formule (I) tel que défini ci-dessus pour la préparation d'un médicament destiné au traitement de pathologies à caractère inflammatoire dans lesquelles le TNFα est impliqué.

L'invention concerne également l'utilisation d'un composé de formule (I) tel que défini ci-dessus pour la préparation d'un médicament destiné au traitement de maladies inflammatoires de la peau, au traitement du psoriasis, de la dermatite atopique ou du rhumatisme psoriasique.

La présente invention a aussi pour objet une composition pharmaceutique destinée notamment au traitement des affections susmentionnées, et qui est caractérisée par le fait qu'elle comprend, dans un support pharmaceutiquement acceptable et compatible avec le mode d'administration retenu pour cette composition, au moins un composé de formule (I). Ce composé de formule (I) peut également se présenter sous une de ses formes énantiomériques ou sous la forme d'un de ses sels pharmaceutiquement acceptables.

On va maintenant donner, à titre d'illustration et sans aucun caractère limitatif, plusieurs exemples de préparation de composés actifs de formule (I) selon l'invention, ainsi que des résultats d'activité biologiques de tels composés.

### EXEMPLES DE REALISATION

Les composés de formule générale (I) sont caractérisés par analyse RMN du proton sur un appareil Advanced 400MHz Bruker.

### Exemple 1 : (S)-3-(4-benzyloxy-benzènesulfonylamino)-N-hydroxy-2-pipéridin-1-yl-propionamide

### 1.1 : (S)-3-tert-butoxycarbonylamino-2-pipéridin-1-yl-propanoate de méthyle

14,4ml (104 mmoles) de triéthylamine sont ajoutés à une solution de 8,0g (31 mmoles) de chlorhydrate de (S)-2-amino-3-tert-butoxycarbonylamino-propanoate de méthyle commercial dans 160ml de tert-butanol puis le milieu réactionnel est agité 30min à 40°C, filtré pour éliminer les sels de triéthylammonium. Au filtrat ainsi obtenu, 6ml (44 mmoles) de 1,5-dibromopentane sont additionnés et le milieu réactionnel est chauffé à 80°C pendant 3 jours. Après filtration de l'insoluble, le filtrat est concentré sous vide. Le résidu brut est purifié par chromatographie sur gel de silice élué avec un mélange heptane/ acétate d'éthyle 70/30. 5,3g (59%) de (S)-3-tert-butoxycarbonylamino-2-piperidin-1-yl-propanoate de méthyle sont obtenus sous forme d'une huile incolore.

### 1.2: dichlorhydrate de (S)-3-amino-2-pipéridin-1-yl-propanoate de méthyle

Une solution de 750mg (2,6 mmoles) de (S)-3-tert-butoxycarbonylamino-2-pipéridin-1-yl-propanoate de méthyle dans 6ml de méthanol et 6ml d' acide chlorhydrique isopropanolique de concentration 5-6N est agitée à 40°C pendant 18h. Après concentration sous vide, le résidu est repris dans l'acétate d'éthyle, filtré, séché sous vide. 670mg (98%) de dichlorhydrate de (S)-3-amino-2-pipéridin-1-yl-propanoate de méthyle sont obtenus sous forme d'un solide blanc.

### 1.3 : sel de sodium de l'acide 4-benzyloxy-benzènesulfonique

64ml (539 mmoles) de bromure de benzyle sont additionnés à une solution de 50g (215 mmoles) de sel de sodium de l'acide 4-hydroxy-benzènesulfonique dihydraté dans 700ml d'isopropanol et 250ml (250 mmoles) d'une solution aqueuse d'hydroxyde de sodium de concentration 1M. Le milieu réactionnel est chauffé à 70°C pendant 20h. Après concentration sous vide de l'isopropanol, le produit précipite et est filtré. 61g (100%) de sel de sodium de l'acide 4-benzyloxy-benzènesulfonique sont obtenus sous forme d'un solide blanc.

### 1.4 : chlorure de 4-benzyloxy-benzènesulfonyle

Une solution de 55ml (639 mmoles) de chlorure d'oxalyle dans 250ml de dichlorométhane est additionnée goutte à goutte à une solution de 61g (213 mmoles) de sel de sodium de l'acide 4-benzyloxy-benzènesulfonique dans 200ml de diméthylformamide, en maintenant la température entre -20°C et -10°C. Après addition, le milieu réactionnel est lentement ramené à température ambiante puis agité pendant 18h, versé sur de la glace et extrait à l'acétate d'éthyle. La phase organique est lavée par de l'eau, par une solution aqueuse saturée de chlorure de sodium et concentrée sous vide. 54g (89%) de chlorure de 4-benzyloxy-benzènesulfonyle sont obtenus sous forme d'un solide blanc.

### 1.5: (S)-3-(4-benzyloxy-benzènesulfonylamino)-2-pipéridin-1-yl-propanoate de méthyle

1,2ml (8,5 mmoles) de triéthylamine puis 800mg (2,8 mmoles) de chlorure de 4-benzyloxy-benzènesulfonyle dans 8ml de dichlorométhane sont ajoutés à une solution de 670mg (2,6 mmoles) du dichlorhydrate de (S)-3-amino-2-pipéridin-1-yl-propanoate de méthyle dans 10ml de dichlorométhane. Le milieu réactionnel est agité à température ambiante pendant 18h, hydrolysé puis dilué par du dichlorométhane. Le produit est extrait au dichlorométhane. La phase organique est lavée par de l'eau puis par une solution aqueuse saturée de chlorure de sodium, séchée sur sulfate de magnésium, filtrée et concentrée sous vide. Le résidu obtenu est purifié par chromatographie sur gel de silice élué avec un mélange heptane/acétate d'éthyle 70/30. 940mg (85%) de (S)-3-(4-benzyloxy-benzènesulfonylamino)-2-pipéridin-1-yl-propanoate de méthyle sont obtenus sous la forme d'un solide blanc.

### 1.6 : acide (S)-3-(4-benzyloxy-benzènesulfonylamino)-2-pipéridin-1-yl-propanoique

1,6ml (1,6 mmoles) d'une solution aqueuse d'hydroxyde de lithium de concentration 1 N sont additionnés à une solution de 450mg (1,1 mmoles) de (S)-3-(4-benzyloxy-benzènesulfonylamino)-2-pipéridin-1-yl- propanoate de méthyle dans 15ml de tetrahydrofuranne et 0,5ml d'eau. Après agitation à température ambiante pendant 18h, le tétrahydrofuranne est évaporé sous vide puis 1,8ml d'une solution aqueuse d'acide acétique de concentration 1 N et 15ml d'eau sont additionnés. Le produit précipite et est filtré. 420mg (98%) d' acide (S)-3-(4-benzyloxy-benzènesulfonylamino)-2-pipéridin-1-yl-propanoique sous la forme d'un solide blanc.

### 1.7: (S)-3-(4-benzyloxy-benzènesulfonylamino)-N-hydroxy-2-pipéridin-1-yl-propionamide:

150mg (1,1 mmoles) de 1-hydroxybenzotriazole et 210mg (1,1 mmoles) du chlorhydrate de 1-éthyl-3-(3-diméthylaminopropyl) carbodiimide sont ajoutés à une solution de 420mg (1,0 mmole) d'acide ((S)-3-(4-benzyloxy-benzènesulfonylamino)-2-pipéridin-1-yl-propanoique dans 15ml de diméthylformamide Le milieu réactionnel est agité 20min puis une solution de 160mg (1,1 mmole) de O-tert-butyldiméthylsilylhydroxylamine dans 3ml de diméthylformamide est additionnée. Le milieu réactionnel est ensuite agité à température ambiante pendant 18h puis hydrolysé par 2ml d'une solution aqueuse saturée d'hydrogénocarbonate de sodium et 2ml d'eau. Après agitation pendant 20min pour compléter la déprotection de l'hydroxamate, le produit est extrait par de l'acétate d'éthyle. Les phases organiques sont lavées par une solution aqueuse saturée d'hydrogénocarbonate de sodium puis par une solution aqueuse saturée de chlorure de sodium, séchées sur sulfate de magnésium, filtrées et concentrées sous vide. Le résidu obtenu est cristallisé dans l'éther isopropylique à chaud puis filtré. Le solide ainsi obtenu est recristallisé dans un mélange heptane/acétate d'éthyle 1/1, filtré puis séché à l'étuve sous vide pendant 4h. 150mg de (S)-3-(4-Benzyloxy-benzenesulfonylamino)-N-hydroxy-2-piperidin-1-yl-propionamide sous la forme d'un solide beige de point de fusion 126°C.
RMN ¹H (δ, DMSO) : 1,24-1,34 (m, 2H); 1,34-1,40 (m, 4H); 2,33-2,37 (m, 4H); 2,70-2,79 (t, J=10,8Hz, 1 H); 2,95-2,99 (m, 2H); 5,19 (s, 2H); 7,19 (d, J=8Hz, 2H); 7,32 (m, 1 H); 7,33-7,48 (m, 5H); 7,74 (d, J=8Hz, 2H); 8,86 (s, 1 H); 10,53 (s, 1 H).

### Exemple 2 : (S)-3-(4-benzyloxy-benzènesulfonylamino)-N-hydroxy-2-pyrrolidin-1-yl-propionamide

### 2.1 : (S)-3-tert-butoxycarbonylamino-2-pyrrolidin-1-yl-propanoate de méthyle

10ml (65 mmoles) de triéthylamine sont ajoutés à une solution de 5g (20 mmoles) de chlorhydrate de (S)-2-amino-3-tert-butoxycarbonylamino-propanoate de méthyle commercial dans 150ml de tert-butanol. Le milieu réactionnel est agité 20min à 40°C puis filtré pour éliminer les sels de triéthylammonium. Au filtrat ainsi obtenu, 3,8ml (32 mmoles) de 1,4-dibromobutane sont ajoutés et le milieu réactionnel est chauffé à 80°C pendant 3 jours. Après filtration de l'insoluble, le filtrat est concentré sous vide. Le résidu obtenu est purifié par chromatographie sur colonne de silice élué avec un mélange dichlorométhane/ méthanol 98/2. 2,9g (55%) de (S)-3-tert-butoxycarbonylamino-2-pyrrolidin-1-yl-propanoate de méthyle sont obtenus sous forme d'une huile jaune.

### 2.2: dichlorhydrate de (S)-3-amino-2-pyrrolidin-1-yl-propanoate de méthyle

Une solution de 2,9g (11 mmoles) de (S)-3-tert-butoxycarbonylamino-2-pyrrolidin-1-yl-propanoate de méthyle dans 20ml d'acide chlorhydrique isopropanolique de concentration 5-6N est agité à 40°C pendant 18h. Après évaporation sols vide, le résidu est repris dans 100ml d'éther diéthylique, agité à température ambiante pendant 1h, filtré et séché sous vide. 2,3g (88%) de dichlorhydrate de (S)-3-amino-2-pyrrolidin-1-yl-propanoate de méthyle sont obtenus sous forme d'un solide beige.

### 2.3: (S)-3-(4-benzyloxy-benzènesulfonylamino)-2-pyrrolidin-1-yl-propanoate de méthyle

De manière analogue à l'exemple 1.5, à partir de 2,3g (9 mmoles) du dichlorhydrate de (S)-3-amino-2-pyrrolidin-1-yl-propanoate de méthyle et de 2,9g (10 mmoles) de chlorure de 4-benzyloxy-benzènesulfonyle (préparé selon l'exemple 1.4), 3,5g (90%) de (S)-3-(4-benzyloxy-benzènesulfonylamino)-2-pyrrolidin-1-yl-propanoate de méthyle sont obtenus sous forme d'un solide blanc.

### 2.4: acide ((S)-3-(4-benzyloxy-benzènesulfonylamino)-2-pyrrolidin-1-yl-propanoique

De manière analogue à l'exemple 1.6, à partir de 500mg (1,2 mmoles) de (S)-3-(4-benzyloxy-benzènesulfonylamino)-2-pyrrolidin-1-yl- propanoate de méthyle, 450mg (94%) d' acide ((S)-3-(4-benzyloxy-benzènesulfonylamino)-2-pyrrolidin-1-yl-propanoique sont obtenus sous forme d'un solide blanc.

### 2.5: (S)-3-(4-benzyloxy-benzènesulfonylamino)-N-hydroxy-2-pyrrolidin-1-yl-propionamide:

De manière analogue à l'exemple 1.7, à partir de 450mg (1,1 mmoles) d'acide (S)-3-(4-benzyloxy-benzènesulfonylamino)-2-pyrrolidin-1-yl-propanoique, 150mg (33%) de (S)-3-(4-benzyloxy-benzènesulfonylamino)-N-hydroxy-2-pyrrolidin-1-yl-propionamide sont obtenus sous forme d'un solide blanc de point de fusion 186°C.
RMN ¹H (δ, DMSO) : 1,50-1,60 (m, 4H); 2,41-2,44 (m, 2H); 2,51-2,54 (m, 2H); 2,77-2,81 (m, 1 H); 2,93-2,99 (m, 1 H); 3,06 (t, J=6,4Hz, 1 H); 5,19 (s, 2H); 7,18 (d, J=8,9Hz, 2H); 7,30-7,33 (m, 1 H); 7,34-7,48 (m, 5H); 7,72 (d, J=8,9Hz, 2H); 8,86 (s, 1 H); 10,61 (s, 1 H).

### Exemple 3 : (S)-3-[4-(4-fluoro-benzyloxy)-benzènesulfonylamino]-N-hydroxy-2-pipéridin-1-yl-propionamide

### 3.1 : (S)-3-(4-hydroxy-benzènesulfonylamino)-2-pipéridin-1-yl-propanoate de méthyle

Une solution de 4,9g (11 mmoles) de (S)-3-(4-benzyloxy-benzènesulfonylamino)-2-pipéridin-1-yl-propanoate de méthyle (préparé comme décrit en 1.5) dans 80ml d'acétate d'éthyle, 30ml de dioxanne et 0,5ml d'acide acétique glacial est dégazée sous azote pendant 15min puis une suspension de 490mg (10% massique) de palladium sur charbon 10% dans 3ml de dioxanne est ajoutée. Le milieu réactionnel est ensuite placé sous une atmosphère d'hydrogène et agité à température ambiante pendant 5h. Après filtration sur célite, le filtrat est concentré sous vide. Le résidu est repris dans de l'acétate d'éthyle, lavé à l'eau, séché sur sulfate de magnésium, filtré et concentré. 3,8g (97%) de (S)-3-(4-hydroxy-benzènesulfonylamino)-2-pipéridin-1-yl-propanoate méthyle sont obtenus sous forme d'un solide blanc.

### 3.2: (S)-3-[4-(4-fluoro-benzyloxy)-benzènesulfonylamino]-2-pipéridin-1-yl-propanoate de méthyle

420mg (1,3 mmoles) de carbonate de césium puis 0,2ml (1,3 mmoles) de 1-bromométhyl-4-fluoro-benzène sont additionnés sur une solution de 400mg (1,2 mmoles) de (S)-3-(4-hydroxy-benzènesulfonylamino)-2-pipéridin-1-yl-propanoate de méthyle dans 10ml d'acétone. Après agitation à température ambiante pendant 18h, le milieu réactionnel est filtré puis le filtrat est concentré sous vide. Le produit brut est purifié par chromatographie sur gel de silice élué avec un mélange heptane/acétate d'éthyle 70/30. 460mg (87%) de (S)-3-[4-(4-fluoro-benzyloxy)-benzènesulfonylamino]-2-pipéridin-1-yl-propanoate de méthyle sont obtenus sous forme d'un solide blanc.

### 3.3: acide (S)-3-[4-(4-fluoro-benzyloxy)-benzènesulfonylamino]-2-pipéridin-1-yl-propanoique

De manière analogue à l'exemple 1.6, à partir de 460mg (1,0 mmole) de (S)-3-[4-(4-fluoro-benzyloxy)-benzènesulfonylamino]-2-pipéridin-1-yl-propanoate, 380mg (86%) d' acide (S)-3-[4-(4-fluoro-benzyloxy)-benzènesulfonylamino]-2-pipéridin-1-yl-propanoique sont obtenus sous forme d'un solide blanc.

### 3.4: (S)-3-[4-(4-fluoro-benzyloxy)-benzènesulfonylamino]-N-hydroxy-2-pipéridin-1-yl-propionamide

De manière analogue à l'exemple 1.7, à partir de 380mg (0,9 mmoles) d'acide (S)-3-[4-(4-fluoro-benzyloxy)-benzènesulfonylamino]-2-pipéridin-1-yl-propanoique, 170mg (43%) de (S)-3-[4-(4-fluoro-benzyloxy)-benzènesulfonylamino]-N-hydroxy-2-pipéridin-1-yl-propionamide sont obtenus sous forme d'un solide blanc de point de fusion 140°C.
RMN ¹H (δ, DMSO) : 1,30-1,34 (m, 2H); 1,35-1,40 (m, 4H); 2,29-2,37 (m, 4H); 2,77-2,79 (m, 1 H); 2,95-2,99 (m, 2H); 5,18 (s, 2H); 7,18-7,27 (m, 4H); 7,34 (s, 1H); 7,50-7,54 (m, 2H); 7,72-7,75 (m, 2H); 8,85 (s, 1 H); 10,53 (s, 1 H).

### Exemple 4 : (S)-3-[(4-benzyloxy-benzènesulfonyl)-méthyl-amino]-N-hydroxy-2-pipéridin-1-yl-propionamide

### 4.1 : (S)-3-[(4-benzyloxy-benzènesulfonyl)-méthyl-amino]-2-pipéridin-1-yl- propanoate de méthyle

190mg (1,4 mmoles) de carbonate de potassium puis 0,15ml ( 2,3 mmoles) d' iodure de méthyle sont additionnés à une solution de 500mg (1,2 mmoles) de (S)-3-(4-benzyloxy-benzènesulfonylamino)-2-pipéridin-1-yl-propanoate de méthyle (préparé comme décrit en 1.5) dans 10ml de diméthylformamide. Le milieu réactionnel est ensuite agité à température ambiante pendant 18h, filtré puis concentré sous vide. Le résidu brut est purifié par chromatographie sur gel de silice élué avec un mélange heptane / acétate d'éthyle 8/2. 400mg (78%) de (S)-3-[(4-benzyloxy-benzènesulfonyl)-méthyl-amino]-2-pipéridin-1-yl- propanoate de méthyle sont obtenus sous forme d'une huile incolore.

### 4.2: acide (S)-3-[(4-benzyloxy-benzènesulfonyl)-méthyl-amino]-2-piperidin-1-yl-propanoique

De manière analogue à l'exemple 1.6, à partir de 400mg (0,9 mmoles) de (S)-3-[(4-benzyloxy-benzènesulfonyl)-méthyl-amino]-2-pipéridin-1-yl-propanoate de méthyle, 350mg (92%) d' acide (S)-3-[(4-benzyloxy-benzènesulfonyl)-méthyl-amino]-2-piperidin-1-yl-propanoique sont obtenus sous forme d'un solide blanc.

### 4.3: (S)-3-[(4-benzyloxy-benzènesulfonyl)-méthyl-amino]-N-hydroxy-2-pipéridin-1-yl-propionamide

De manière analogue à l'exemple 1.7, à partir de 350mg (0,8 mmoles) d'acide (S)-3-[(4-benzyloxy-benzènesulfonyl)-méthyl-amino]-2-pipéridin-1-yl-propanoique, 30mg (8%) de (S)-3-[(4-benzyloxy-benzènesulfonyl)-méthyl-amino]-N-hydroxy-2-pipéridin-1-yl-propionamide sont obtenus sous forme d'un solide crème de point de fusion 102°C.
RMN ¹H (δ, DMSO) : 1,33-1,34 (m, 2H); 1,41-1,43 (m, 4H); 2,41-2,43 (m, 2H); 2,52-2,54 (m, 2H); 2,65 (s, 3H); 3,02-3,09 (m, 1H); 3,13-3,19 (m, 2H); 5,21 (s, 2H); 7,23 (d, J=8,8Hz, 2H); 7,34-7,43 (m, 3H); 7,47 (d, J=7Hz, 2H); 7,71 (d, J=8.8Hz, 2H); 8,91 (s, 1 H); 10,59 (s, 1 H).

### Exemple 5 : (S)-N-Hydroxy-3-[4-(naphthalen-2-ylméthoxy)-benzènesulfonylamino]-2-pipéridin-1-yl-propionamide

### 5.1 : (S)-3-[4-(naphthalen-2-ylméthoxy)-benzènesulfonylamino]-2-pipéridin-1-yl-propanoate de méthyle

420mg (1,3 mmoles) de carbonate de césium suivis de 280mg (1,3 mmoles) de 2-bromométhyl-naphthalène sont additionnés à une solution de 400mg (1,2 mmoles) de (S)-3-(4-hydroxy-benzènesulfonylamino)-2-pipéridin-1-yl-propanoate de méthyle (préparé comme décrit en 3.1) dans 10ml d'acétone. Le milieu réactionnel est agité à température ambiante pendant 18h puis filtré. Le filtrat est concentré sous vide et purifié par chromatographie sur gel de silice élué avec un mélange heptane/acétate d'éthyle 70/30. 420mg (80%) de (S)-3-[4-(naphthalen-2-ylméthoxy)-benzènesulfonylamino]-2-pipéridin-1-yl-propanoate de méthyle sont obtenus sous forme d'un solide blanc.

### 5.2 : acide (S)-3-[4-(Naphthalen-2-ylméthoxy)-benzènesulfonylamino]-2-pipéridin-1-yl-propanoique

De manière analogue à l'exemple 1.6, à partir de 420mg (0,9 mmoles) de (S)-3-[4-(naphthalen-2-ylméthoxy)-benzènesulfonylamino]-2-pipéridin-1-yl-propanoate de méthyle, 370mg (90%) d' acide (S)-3-[4-(naphthalen-2-ylméthoxy)-benzènesulfonylamino]-2-pipéridin-1-yl-propanoique sont obtenus sous forme d'un solide blanc.

### 5.3: (S)-N-hydroxy-3-[4-(naphthalen-2-ylméthoxy)-benzènesulfonylamino]-2-pipéridin-1-yl-propionamide

De manière analogue à l'exemple 1.7, à partir de 370mg (0,8 mmoles) d'acide (S)-3-[4-(naphthalen-2-ylméthoxy)-benzènesulfonylamino]-2-pipéridin-1-yl-propanoique, 30mg (8%) de (S)-N-hydroxy-3-[4-(naphthalen-2-ylméthoxy)-benzènesulfonylamino]-2-pipéridin-1-yl-propionamide sont obtenus sous forme d'un solide beige de point de fusion 106°C.
RMN ¹H (δ, DMSO) : 1,28-1,30 (m, 2H); 1,35-1,45 (m, 4H); 2,30-2,40 (m, 4H); 2,76 -2,81 (m, 1 H); 2,95-2,97 (m, 1 H); 2-97-3,01 (1 H); 5,38 (s, 2H); 7,24 (d, J=8,9Hz, 2H); 7,33 (m, 1 H); 7,53-7,56 (m, 2H); 7,59 (d, J=8.4Hz, 1 H); 7,75 (d, J=8,8Hz, 2H); 7,93-7,97 (m, 3H); 8,01 (s, 1 H); 8,85 (s, 1 H); 10,53 (s, 1 H).

### Exemple 6 : (S)-3-[4-(3,4-dichloro-benzyloxy)-benzènesulfonylamino]-N-hydroxy-2-pipéridin-1-yl-propionamide

### 6.1 : (S)-3-[4-(3,4-dichloro-benzyloxy)-benzènesulfonylamino]-2-pipéridin-1-yl-propanoate de méthyle

De manière analogue à l'exemple 5.1, à partir de 310mg (1,3 mmoles) de 4-bromométhyl-1,2-dichloro-benzène et de 400mg (1,2 mmoles) de (S)-3-(4-hydroxy-benzènesulfonylamino)-2-pipéridin-1-yl-propanoate de méthyle (préparé comme décrit en 3.1), 460mg (78%) de (S)-3-[4-(3,4-dichloro-benzyloxy)-benzènesulfonylamino]-2-pipéridin-1-yl-propanoate de méthyle sont obtenus sous forme d'un solide blanc.

### 6.2: acide ((S)-3-[4-(3,4-dichloro-benzyloxy)-benzènesulfonylamino]-2-piperidin-1-yl-propanoique

De manière analogue à l'exemple 1.6, à partir de 460mg (0,9 mmoles) de ((S)-3-[4-(3,4-dichloro-benzyloxy)-benzènesulfonylamino]-2-pipéridin-1-yl-propanoate de méthyle, 420mg (93%) d' acide ((S)-3-[4-(3,4-dichloro-benzyloxy)-benzènesulfonylamino]-2-pipéridin-1-yl-propanoique sont obtenus sous forme d'un solide blanc.

### 6.3 : (S)-3-[4-(3,4-dichloro-benzyloxy)-benzènesulfonylaminol-N-hydroxy-2-pipéridin-1-yl-propionamide

De manière analogue à l'exemple 1.7, à partir de 420mg (0,9 mmoles) d' acide ((S)-3-[4-(3,4-dichloro-benzyloxy)-benzènesulfonylamino]-2-pipéridin-1-yl-propanoique, 240mg (47%) de (S)-3-[4-(3,4-dichloro-benzyloxy)-benzènesulfonylamino]-N-hydroxy-2-pipéridin-1-yl-propionamide sont obtenus sous forme d'un solide blanc de point de fusion 166°C.
RMN ¹H (δ, DMSO) : 1,18-1,23 (m, 2H); 1,23-1,32 (m, 4H); 2,20-2,35 (m,4H); 2,66-2,72 (m, 1 H); 2,87-2,89 (m, 2H); 5,68 (s, 2H); 7,12 (d, J=8,8Hz, 2H); 7,27 (s, 1 H); 7.39 (d, J=8,2Hz, 1 H); 7,60 (d, J= 8,2Hz, 1 H); 7,67 (d, J=8,6Hz, 3H); 8,78 (s, 1 H); 10,46 (s, 1 H).

### Exemple 7 : (S)-3-(4-benzyloxy-benzènesulfonylamino)-N-hydroxy-2-morpholin-4-yl-propionamide

### 7.1 : (S)-3-tert-butoxycarbonylamino-2-morpholin-4-yl-propanoate de méthyle

2,8 g (20 mmoles) de 1-chloro-2-(2-chloro-éthoxy)-éthane sont ajoutés à une solution de 5g (20 mmoles) de (S)-2-amino-3-tert-butoxycarbonylamino-propanoate de méthyle commercial dans 65ml de diisopropyléthylamine. Le mélange réactionnel est agité à 127°C pendant 18h. Après addition d'eau, le milieu réactionnel est extrait à l'acétate d'éthyle. Les phases organiques sont rassemblées, séchées sur sulfate de sodium, filtrées, évaporées. Le résidu obtenu est purifié par chromatographie sur gel de silice élué avec un mélange heptane/acétate d'éthyle 70/30. 1,4g (24%) de (S)-3-tert-butoxycarbonylamino-2-morpholin-4-yl-propanoate de méthyle sont obtenus sous forme d'une huile.

### 7.2 : (S)-3-amino-2-morpholin-4-yl-propanoate de méthyle

Une solution de 1,4g (5 mmoles) de (S)-3-tert-butoxycarbonylamino-2-morpholin-4-yl-propanoate de méthyle dans 20ml de méthanol et 10ml d'une solution d'acide chlorhydrique de concentration 5-6N dans l'isopropanol est agitée à 40°C pendant 20h puis évaporée à sec. 1,3g (96%) de dichlorhydrate de (S)-3-amino-2-morpholin-4-yl-propanoate de méthyle sont obtenus sous forme d'un solide.

### 7.3 : (S)-3-(4-benzyloxy-benzènesulfonylamino)-2-morpholin-4-yl-propanoate de méthyle

Une solution de 1,5g (5,3 mmoles) de chlorure de 4-benzyloxy-benzènesulfonyle (préparé selon l'exemple 1.4) dans 20ml de dichlorométhane est additionnée goutte à goutte à une solution de 1,3g (4,8 mmoles) de dichlorhydrate de (S)-3-amino-2-morpholin-4-yl-propanoate de méthyle, 2,7ml (19,3 mmoles) de triéthylamine dans 40ml de dichlorométhane. Le mélange réactionnel est agité à température ambiante pendant 18h. Après addition d'eau et extraction au dichlorométhane, les phases organiques sont rassemblées, séchées sur sulfate de sodium, évaporées. Le résidu est purifié par chromatographie sur gel de silice élué avec un mélange heptane/acétate d'éthyle 60/40.
1,8g (87%) de (S)-3-(4-benzyloxy-benzènesulfonylamino)-2-morpholin-4-yl-propanoate de méthyle sont obtenus sous forme d'un solide blanc.

### 7.4 : acide (S)-3-(4-benzyloxy-benzènesulfonylamino)-2-morpholin-4-yl-propanoique

De manière analogue à l'exemple 1.6, à partir de 500mg (1,2 mmoles) de (S)-3-(4-benzyloxy-benzènesulfonylamino)-2-morpholin-4-yl-propanoate de méthyle, 434mg (90%) d'acide (S)-3-(4-benzyloxy-benzènesulfonylamino)-2-morpholin-4-yl-propanoique sont obtenus sous forme d'un solide blanc.

### 7.5: (S)-3-(4-benzyloxy-benzènesulfonylamino)-N-hydroxy-2-morpholin-4-yl-propionamide

De manière analogue à l'exemple 1.7, à partir de 429mg (1,0 mmoles) d'acide (S)-3-(4-benzyloxy-benzènesulfonylamino)-2-morpholin-4-yl-propanoique, 12mg (11%) de (S)-3-(4-benzyloxy-benzènesulfonylamino)-N-hydroxy-2-morpholin-4-yl-propionamide sont obtenus sous forme d'une poudre beige de point de fusion 142°C.
RMN ¹H (δ, DMSO) : 2,35-2,45 (m, 4H); 2,79-2,86 (m, 1 H); 2,93-3,01 (m, 2H); 3,40-3,55 (m, 4H); 5,20 (s, 2H); 7,19 (d, J=8,8Hz, 2H); 7,34-7,48 (m, 6H); 7,74 (d, J=8,7Hz, 2H); 8,92 (s, 1H); 10,63 (s, 1 H).

### Exemple 8 : (S)-N-hydroxy-3-[4-(2-méthyl-quinolin-4-ylméthoxy)-benzènesulfonylamino]-2-pipéridin-1-yl-propionamide

### 8.1 : (S)-3-[4-(2-méthyl-quinolin-4-ylméthoxy)-benzènesulfonylamino]-2-pipéridin-1-yl-propanoate de méthyle

De manière analogue à l'exemple 5.1, à partir de 240mg (1,3 mmoles) de 4-chlorométhyl-2-méthyl-quinoline et de 400mg (1,2 mmoles) de (S)-3-(4-hydroxy-benzènesulfonylamino)-2-pipéridin-1-yl-propanoate de méthyle (préparé comme décrit en 3.1), 450mg (77%) de (S)-3-[4-(2-méthyl-quinolin-4-ylméthoxy)-benzènesulfonylamino]-2-pipéridin-1-yl-propanoate de méthyle sont obtenus sous forme d'un solide blanc.

### 8.2 : acide (S)-3-[4-(2-méthyl-quinolin-4-ylméthoxy)-benzènesulfonylamino]-2-pipéridin-1-yl-propanoique

De manière analogue à l'exemple 1.6, à partir de 450mg (0,9 mmoles) de (S)-3-[4-(2-méthyl-quinolin-4-ylméthoxy)-benzènesulfonylamino]-2-pipéridin-1-yl-propanoate de méthyle, 360mg (84%) d' acide (S)-3-[4-(2-méthyl-quinolin-4-ylméthoxy)-benzènesulfonylamino]-2-pipéridin-1-yl-propanoique sont obtenus sous forme d'un solide blanc.

### 8.3: (S)-N-hydroxy-3-[4-(2-méthyl-quinolin-4-ylméthoxy)-benzènesulfonylamino]-2-pipéridin-1-yl-propionamide

De manière analogue à l'exemple 1.7, à partir de 360mg (0,7 mmoles) d'acide (S)-3-[4-(2-méthyl-quinolin-4-ylméthoxy)-benzènesulfonylamino]-2-pipéridin-1-yl-propanoique, 50mg (13%) de (S)-N-hydroxy-3-[4-(2-méthyl-quinolin-4-ylméthoxy)-benzènesulfonylamino]-2-pipéridin-1-yl-propionamide sont obtenus sous forme d'un solide blanc de point de fusion 150°C.
RMN ¹H (δ, DMSO) : 1,29-1,31 (m, 2H); 1,39-1,40 (m, 4H); 2,34-2,38 (m, 4H); 2,67 (s, 3H); 2,73-2,79 (m, 1 H); 2,96-2,99 (m, 2H); 5,72 (s, 2H); 7,33-7,37 (m, 3H); 7,57-7,62 (m, 2H); 7,74-7,80 (m, 1 H); 7,98 (d, J=8,4Hz, 2H); 8,11 (d, J=8,4Hz, 2H); 8,86 (s, 1 H), 10,54 (s, 1 H).

### Exemple 9 : (S)-3-[4-(3,5-dichloro-benzyloxy)-benzènesulfonylamino]-N-hydroxy-2-pipéridin-1-yl-propionamide

### 9.1 : (S)-3-[4-(3,5-dichloro-benzyloxy)-benzènesulfonylamino]-2-pipéridin-1-yl-propanoate de méthyle

De manière analogue à l'exemple 5.1, à partir de 240mg (1,3 mmoles) de 4-chlorométhyl-2-méthyl-quinoline et de 400mg (1,2 mmoles) de (S)-3-(4-hydroxy-benzènesulfonylamino)-2-pipéridin-1-yl-propanoate de méthyle (préparé comme décrit en 3.1), 400mg (69%) de (S)-3-[4-(3,5-dichloro-benzyloxy)-benzènesulfonylamino]-2-pipéridin-1-yl-propanoate de méthyle sont obtenus sous forme d'un solide blanc.

### 9.2 : acide-(S)-3-[4-(3,5-dichloro-benzyloxy)-benzènesulfonylamino]-2-pipéridin-1-yl-propanoique

De manière analogue à l'exemple 1.6, à partir de 400mg (0,8 mmoles) de (S)-3-[4-(3,5-dichloro-benzyloxy)-benzènesulfonylamino]-2-pipéridin-1-yl-propanoate de méthyle, 360mg (92%) d' acide (S)-3-[4-(3,5-dichloro-benzyloxy)-benzènesulfonylamino]-2-pipéridin-1-yl-propanoique sont obtenus sous forme d'un solide blanc.

### 9.3: (S)-3-[4-(3,5-dichloro-benzyloxy)-benzènesulfonylamino]-N-hydroxy-2-pipéridin-1-yl-propionamide

De manière analogue à l'exemple 1.7, à partir de 360mg ( 0,7 mmoles) d'acide (S)-3-[4-(3,5-dichloro-benzyloxy)-benzènesulfonylamino]-2-pipéridin-1-yl-propanoique, 80mg (22%) de (S)-3-[4-(3,5-dichloro-benzyloxy)-benzènesulfonylamino]-N-hydroxy-2-pipéridin-1-yl-propionamide sont obtenus sous forme d'un solide jaune de point de fusion 130°C.
RMN ¹H (δ, DMSO) : 1,28-1,35 (m, 2H); 1,35-1,42 (m, 4H); 2,31-2,40 (m, 4H); 2-75-2,82 (m, 1 H); 2,94-3,00 (m, 2H); 5,23 (s, 2H); 7,20 (d, J=8,9Hz, 2H); 7,30-7,40 (m, 1 H); 7,54 (s, 2H); 7,60 (s, 1 H); 7,75 (d, J=8,5Hz, 2H); 8,85 (s, 1 H); 10,54 (s, 1 H).

### Exemple 10 : (S)-N-hydroxy-2-pipéridin-1-yl-3-(4-propoxy-benzènesulfonylamino)-propionamide

### 10.1 : (S)-2-pipéridin-1-yl-3-(4-propoxy-benzènesulfonylamino)-propanoate de méthyle

De manière analogue à l'exemple 5.1, à partir de 0,1 ml (1,5 mmoles) de 1-bromopropane et de 400mg (1,2 mmoles) de (S)-3-(4-hydroxy-benzènesulfonylamino)-2-pipéridin-1-yl-propanoate de méthyle (préparé comme décrit en 3.1), 240mg (53%) de (S)-2-pipéridin-1-yl-3-(4-propoxy-benzènesulfonylamino)-propanoate de méthyle sont obtenus sous forme d'une huile incolore.

### 10.2 : acide (S)-2-pipéridin-1-yl-3-(4-propoxy-benzènesulfonylamino)-propanoique

De manière analogue à l'exemple 1.6, à partir de 240mg (0,6 mmoles) de (S)-2-pipéridin-1-yl-3-(4-propoxy-benzènesulfonylamino)-propanoate de méthyle, 180mg (78%) d' acide (S)-2-pipéridin-1-yl-3-(4-propoxy-benzènesulfonylamino)-propanoique sont obtenus sous forme d'un solide blanc.

### 10.3: (S)-N-hydroxy-2-pipéridin-1-yl-3-(4-propoxy-benzènesulfonylamino)-propionamide

De manière analogue à l'exemple 1.7, à partir de 180mg (0,5 mmoles) d'acide (S)-2-pipéridin-1-yl-3-(4-propoxy-benzènesulfonylamino)-propanoique, 50mg (27%) de (S)-N-hydroxy-2-pipéridin-1-yl-3-(4-propoxy-benzènesulfonylamino)-propionamide sont obtenus sous forme d'un solide blanc de point de fusion 147°C.
RMN ¹H (δ, DMSO) : 0,91 (t, J=7,3Hz, 3H); 1,20-1,26 (m, 2H); 1,30-1,40 (m, 4H); 1,63-1,72 (m, 2H); 2,25-2,35 (m, 4H); 2,65-2,75 (m, 1 H); 2,85-2,95 (m, 2H); 3,94 (t, J=6,4Hz, 2H); 7,02 (d, J=8,7Hz, 2H); 7,25 (s, 1 H); 7,65 (d, J=8,7Hz, 2H); 8,78 (s, 1 H); 10,42 (s, 1 H).

### Exemple 11 : (S)-3-(4-cyclopropylméthoxy-benzènesulfonylamino)-N-hydroxy-2-morpholin-4-yl-propionamide

### 11.1 : (S)-3-(4-hydroxy-benzènesulfonylamino)-2-morpholin-4-yl-propanoate de méthyle

0,4g de palladium sur charbon 10% sont ajoutés à une solution de 4g (9,2 mmoles) de (S)-3-(4-benzyloxy-benzènesulfonylamino)-2-morpholin-4-yl-propanoate de méthyle (préparé comme décrit dans l'exemple 7.3) dans 200ml de méthanol et 20ml de dioxanne préalablement dégazée à l'azote. Le mélange réactionnel est agité une heure à température ambiante sous pression atmosphérique d'hydrogène. Après filtration sur célite et concentration sous vide du filtrat, le résidu obtenu est purifié par chromatographie sur gel de silice élué avec un mélange acétate d'éthyle/heptane 60/40. 2,6g (81%) de (S)-3-(4-hydroxy-benzènesulfonylamino)-2-morpholin-4-yl-propanoate de méthyle sont obtenus sous forme d'un solide blanc.

### 11.2 : (S)-3-(4-cyclopropylméthoxy-benzènesulfonylamino)-2-morpholin-4-yl-propanoate de méthyle

De manière analogue à l'exemple 5.1, à partir de 173mg (1,3 mmoles) de bromure de cyclopropyl méthyle et de 400mg (1,2 mmoles) de (S)-3-(4-hydroxy-benzènesulfonylamino)-2-morpholin-4-yl-propanoate de méthyle, 272mg (59%) de (S)-3-(4-cyclopropylméthoxy-benzènesulfonylamino)-2-morpholin-4-yl-propanoate de méthyle sont obtenus sous forme d'une huile.

### 11.3 : acide (S)-3-(4-cyclopropylméthoxy-benzènesulfonylamino)-2-morpholin-4-yl-propanoique

De manière analogue à l'exemple 1.6, à partir de 262mg (0,7 mmoles) de (S)-3-(4-cyclopropylméthoxy-benzènesulfonylamino)-2-morpholin-4-yl-propanoate de méthyle, 197mg (78%) d'acide (S)-3-(4-cyclopropylméthoxy-benzènesulfonylamino)-2-morpholin-4-yl-propanoique sont obtenus sous forme d'une poudre blanche.

### 11.4 : (S)-3-(4-cyclopropylméthoxy-benzènesulfonylamino)-N-hydroxy-2-morpholin-4-yl-propionamide

De manière analogue à l'exemple 1.7, à partir de 192mg (0,5 mmoles) d'acide (S)-3-(4-cyclopropylméthoxy-benzènesulfonylamino)-2-morpholin-4-yl-propanoique, 88mg (44%) de (S)-3-(4-cyclopropylméthoxy-benzènesulfonylamino)-N-hydroxy-2-morpholin-4-yl-propionamide sont obtenus sous forme d'une poudre blanche de point de fusion 147°C.
RMN ¹H (δ, DMSO) : 0,82-0,90 (m, 2H); 0,58-0,68 (m, 2H); 1,25-1,35 (m, 1 H); 2,43-2,41 (m, 4H); 2,80-2,90 (m, 1 H); 2,90-2,98 (m, 1 H); 2,98-3,05 (m, 1 H); 3,48-3,58 (m, 4H); 3,94 (d, J=7Hz, 2H); 7,13 (d, J=8,9Hz, 2H); 7,47 (m, 1H); 7,75 (d, J=8,8Hz, 2H); 8,95 (s, 1H); 10,67 (s, 1 H).

### Exemple 12 : (S)-3-[4-(4-tert-butyl-benzyloxy)-benzènesulfonylamino]-N-hydroxy-2-morpholin-4-yl-propionamide

### 12.1: (S)-3-[4-(4-tert-butyl-benzyloxy)-benzènesulfonylamino]-2-morpholin-4-yl-propanoate de méthyle

De manière analogue à l'exemple 5.1, à partir de 290mg (1,3 mmoles) de bromure de 4-tert butyl-benzyle et de 400mg (1,2 mmoles) de (S)-3-(4-hydroxy-benzènesulfonylamino)-2-morpholin-4-yl-propanoate de méthyle (préparé comme décrit dans l'exemple 11.1), 434mg (76%) de (S)-3-[4-(4-tert-butyl-benzyloxy)-benzènesulfonylamino]-2-morpholin-4-yl-propanoate de méthyle sont obtenus sous forme d'une poudre blanche.

### 12.2: acide (S)-3-[4-(4-tert-butyl-benzyloxy)-benzènesulfonylamino]-2-morpholin-4-yl-propanoique

De manière à l'exemple 1.6, à partir de 429mg (0,9 mmoles) de (S)-3-(4-cyclopropylméthoxy-benzènesulfonylamino)-2-morpholin-4-yl-propanoate de méthyle, 359mg (86%) d'acide (S)-3-[4-(4-tert-butyl-benzyloxy)-benzènesulfonylamino]-2-morpholin-4-yl-propanoique sont obtenus sous forme d'une poudre blanche.

### 12.3 : (S)-3-[4-(4-tert-butyl-benzyloxy)-benzènesulfonylamino]-N-hydroxy-2-morpholin-4-yl-propionamide

De manière analogue à l'exemple 1.7, à partir de 355mg (0,8 mmoles) d'acide (S)-3-[4-(4-tert-butyl-benzyloxy)-benzènesulfonylamino]-2-morpholin-4-yl-propanoique, 277mg (76%) de (S)-3-[4-(4-tert-butyl-benzyloxy)-benzènesulfonylamino]-N-hydroxy-2-morpholin-4-yl-propionamide sont obtenus sous forme d'une poudre beige de point de fusion 137°C.
RMN ¹H (δ, DMSO) : 1,28 (s, 9H); 2,40 (m, 4H); 2,79-2,83 (m, 1 H); 2,93-3,01 (m, 2H); 3,46 (m, 4H); 5,15 (s, 2H); 7,18 (d, J=8,8Hz, 2H); 7,40 (q, J= 8,4Hz, 5H); 7,74 (d, J=8,8Hz, 2H); 8,92 (s, 1 H); 10,63 (s, 1 H).

### Exemple 13 : (S)-N-hydroxy-2-morpholin-4-yl-3-(4-phénéthyloxy-benzènesulfonylamino)-propionamide

### 13.1 : (S)-2-morpholin-4-yl-3-(4-phénéthyloxy-benzènesulfonylamino)-propanoate de méthyle

De manière analogue à l'exemple 5.1, à partir de 237mg (1,3 mmoles) de 2-bromoéthylbenzène et de 400mg (1,2 mmoles) de (S)-3-(4-hydroxy-benzènesulfonylamino)-2-morpholin-4-yl-propanoate de méthyle (préparé comme décrit en 11.1), 215mg (41%) de (S)-2-morpholin-4-yl-3-(4-phénéthyloxy-benzènesulfonylamino)-propanoate de méthyle sont obtenus sous forme d'une huile incolore.

### 13.2 : acide (S)-2-morpholin-4-yl-3-(4-phénéthyloxy-benzènesulfonylamino)-propanoique

De manière analogue à l'exemple 1.6, à partir de 210mg (0,5 mmoles) de (S)-2-morpholin-4-yl-3-[4-(pyridin-4-ylméthoxy)-benzènesulfonylamino]-propanoate de méthyle, 155mg (76%) d'acide (S)-2-morpholin-4-yl-3-(4-phénéthyloxy-benzènesulfonylamino)-propanoique sont obtenus sous forme d'une poudre blanche.

### 13.3 : (S)-N-hydroxy-2-morpholin-4-yl-3-(4-phénéthyloxy-benzènesulfonylamino)-propionamide

De manière analogue à l'exemple 1.7, à partir de 170mg (0,4 mmoles) d'acide (S)-2-morpholin-4-yl-3-(4-phénéthyloxy-benzènesulfonylamino)-propanoique, 72mg (46%) de (S)-N-hydroxy-2-morpholin-4-yl-3-(4-phénéthyloxy-benzènesulfonylamino)-propionamide sont obtenus sous forme d'une poudre beige de point de fusion 77°C.
RMN ¹H (δ, DMSO) : 2,35-2,42 (m, 4H); 2,75-2,85 (m, 1 H); 2,90-3,01 (m, 2H); 3,06 (t, J=6,8Hz, 2H); 3,40-3,50 (m, 4H); 4,28 (t, J=6,8Hz, 2H); 7,11 (d, J=8,9Hz, 2H); 7,25 (m, 1 H); 7,32 (m, 4H); 7,45 (m, 1 H); 7,71 (d, J=8,8Hz, 2H); 8,91 (s, 1 H); 10,62 (s, 1 H).

### Exemple 14 : (S)-N-hydroxy-3-[4-(2-méthyl-quinolin-4-ylméthoxy)-benzènesulfonylamino]-2-morpholin-4-yl-propionamide

### 14.1 : (S)-3-[4-(2-méthyl-quinolin-4-ylméthoxy)-benzènesulfonylamino]-2-morpholin-4-yl-propanoate de méthyle

De manière analogue à l'exemple 5.1, à partir de 245mg (1,3 mmoles) de 4-chlorométhyl-2-méthylquinoline et de 400mg (1,2 mmoles) de (S)-3-(4-hydroxy-benzènesulfonylamino)-2-morpholin-4-yl-propanoate de méthyle (préparé comme décrit dans l'exemple 11.1), 483mg (83%) de (S)-3-[4-(2-méthyl-quinolin-4-ylméthoxy)-benzènesulfonylamino]-2-morpholin-4-yl-propanoate de méthyle sont obtenus sous forme d'une huile jaune.

### 14.2 : acide (S)-3-[4-(2-méthyl-quinolin-4-ylméthoxy)-benzènesulfonylamino]-2-morpholin-4-yl-propanoique

De manière analogue à l'exemple 1.6, à partir de 479mg (1,0 mmole) de (S)-3-[4-(2-méthyl-quinolin-4-ylméthoxy)-benzènesulfonylamino]-2-morpholin-4-yl-propanoate de méthyle, 397mg (85%) d' acide (S)-3-[4-(2-méthyl-quinolin-4-ylméthoxy)-benzènesulfonylamino]-2-morpholin-4-yl-propanoique sont obtenus sous forme d'une poudre beige.

### 14.3 : (S)-N-hydroxy-3-[4-(2-méthyl-quinolin-4-ylméthoxy)-benzènesulfonylamino]-2-morpholin-4-yl-propionamide

De manière analogue à l'exemple 1.7, à partir de 293mg (0,5 mmoles) d'acide (S)-3-[4-(2-méthyl-quinolin-4-ylméthoxy)-benzènesulfonylamino]-2-morpholin-4-yl-propanoique, 121 mg (40%) de (S)-N-hydroxy-3-[4-(2-méthyl-quinolin-4-ylméthoxy)-benzènesulfonylamino]-2-morpholin-4-yl-propionamide sont obtenus sous forme d'une poudre beige de point de fusion 138°C.
RMN ¹H (δ, DMSO): 2,39-2,42 (m, 4H); 2,67 (s, 3H); 2,82-2,88 (m, 1 H); 2,94-3,02 (m, 2H); 3,45-3,52 (m, 4H); 5,72 (s, 2H); 7,34 (d, J=8,9Hz, 2H); 7,49 (m, 1 H); 7,57-7,62 (m, 2H); 7,74-7,80 (m, 3H); 7,98 (d, J=8Hz, 1 H); 8,11 (d, J=7,8Hz, 1 H); 8,92 (s, 1 H); 10,63 (s, 1 H).

### Exemple 15 : dichlorhydrate de (S)-N-hydroxy-3-[4-(2-méthyl-quinolin-4-ylméthoxy)-benzènesulfonylamino]-2-pipéridin-1-yl-propionamide

0,4ml (2,0 mmoles) d'une solution d'acide chlorhydrique isopropanolique de concentration 5-6N sont ajoutés à une solution de 400mg (0,8 mmoles) de (S)-N-hydroxy-3-[4-(2-méthyl-quinolin-4-ylméthoxy)-benzènesulfonylamino]-2-pipéridin-1-yl-propionamide (préparé comme décrit dans l'exemple 8) dans 8ml d'isopropanol. Le milieu réactionnel est agité à température ambiante pendant 2h puis filtré. Le résidu obtenu est recristallisé dans un mélange isopropanol / eau 10/1, filtré et séché sous vide. 260mg (56%) de dichlorhydrate de (S)-N-hydroxy-3-[4-(2-méthyl-quinolin-4-ylméthoxy)-benzènesulfonylamino]-2-pipéridin-1-yl-propionamide sont obtenus sous forme d'un solide blanc de point de fusion 188°C.
RMN ¹H (δ, DMSO) : 1,30-1,40 (m, 1 H); 1,60-1,80 (m, 4H); 1,85-1,95 (m, 1 H); 2,85 (m, 1 H); 3,00 (s, 3H); 3,10 (m, 1 H); 3,25-3,35 (m, 3H); 3,45 (m, 1 H); 3,80 (m, 1 H); 5,98 (s, 2H); 7,45 (d, J=8,8Hz, 2H); 7,85-7,94 (m, 3H); 8,05-8,10 (m, 1H); 8,10-8,15 (m, 2H); 8,44 (d, J=8Hz, 2H), 11,03 (s, 1 H), 11,54 (s, 1 H).

### Exemple 16 : (R)-N-Hydroxy-3-[4-(2-methyl-quinolin-4-ylmethoxy)-benzenesulfonylamino]-2-piperidin-1-yl-propionamide

### 16.1 : (R)-3-tert-butoxycarbonylamino-2-pipéridin-1-yl-propanoate de méthyle

3,1 ml (20,6 mmoles) de 1,5-diiodopentane sont ajoutés à une solution de 5,0g (19,6 mmoles) du chlorhydrate de (R)-2-amino-3-tert-butoxycarbonylamino-propanoate de méthyle commercial, 0,22g (0,6 mmoles) d'iodure de tétrabutylammonium dans 50ml de N,N-diisopropyléthylamine. Le milieu réactionnel est chauffé à 127°C pendant 5h puis à 100°C pendant 18h. Après évaporation d'un maximun de N,N-diisopropyléthylamine, le milieu réactionnel est hydrolysé puis dilué par de l'acétate d'éthyle. La phase organique est lavée par de l'eau, séchée sur sulfate de magnésium, filtrée et concentrée sous vide.
Le résidu obtenu est purifié par chromatographie sur gel de silice élué avec un mélange heptane/acétate d'éthyle 70/30. 3,7g (66%) de (R)-3-tert-butoxycarbonylamino-2-pipéridin-1-yl-propanoate de méthyle sont obtenus sous forme d'une huile claire.

### 16.2 : dichlorhydrate de (R)-3-amino-2-pipéridin-1-yl-propanoate de méthyle

3,7g (12,9 mmoles) de (R)-3-tert-butoxycarbonylamino-2-pipéridin-1-yl-propanoate de méthyle sont placés dans un mélange de 25ml de méthanol et de 15ml d' acide chlorhydrique en solution dans l'isopropanol de concentration 5-6N . Le milieu réactionnel est agité à 40°C pendant 18h puis concentré sous vide. 3,5g (100%) du dichlorhydrate de (S)-3-amino-2-pipéridin-1-yl-propanoate de méthyle sont obtenus sous forme d'un solide beige.

### 16.3 : sel de sodium de l'acide 4-(2-méthyl-quinolin-4-ylméthoxy)-benzènesulfonique

100g (438 mmoles) du chlorhydrate de 4-chlorométhyl-2-méthyl-quinoline sont additionnés sur une solution de 77g (395 mmoles) du sel de sodium de l'acide 4-hydroxy-benzènesulfonique et de 84ml (84 mmoles) d'une solution aqueuse d'hydroxyde de sodium de concentration 1 M dans 800ml d'isopropanol. Le milieu réactionnel est chauffé à 70°C pendant 5h puis à 40°C pendant 18h.
Après évaporation de l'isopropanol, le produit obtenu est filtré, rincé à l'isopropanol et à l'éther diéthylique puis séché sous vide. 114g (75%) du sel de sodium de l'acide 4-(2-méthyl-quinolin-4-ylméthoxy)-benzènesulfonique sont obtenus sous forme d'un solide blanc.

### 16.4 : chlorure de 4-(2-méthyl-quinolin-4-ylméthoxy)-benzènesulfonyle

76g (216 mmoles) du sel de sodium de l'acide 4-(2-méthyl-quinolin-4-ylméthoxy)-benzènesulfonique dans 500ml de diméthylformamide sont additionnés goutte à goutte à une solution de 55ml (649 mmoles) de chlorure d'oxalyle dans 100ml de dichlorométhane, préalablement refroidie à -10°C. Après addition, le milieu réactionnel est agité à température ambiante pendant 18h. Le milieu réactionnel est ensuite versé dans 1l de glace puis extrait à l'acétate d'éthyle. Les phases organiques sont rassemblées, lavées à l'eau puis avec une solution aqueuse saturée de chlorure de sodium, séchées de sulfate de magnésium, filtrées et concentrées sous vide. 77g (92%) du chlorhydrate de chlorure de 4-(2-méthyl-quinolin-4-ylméthoxy)-benzènesulfonyle sont obtenus sous forme d'un solide beige.

### 16.5 : (R)-3-[4-(2-méthyl-quinolin-4-ylméthoxy)-benzènesulfonylamino]-2-pipéridin-1-yl-propanoate de méthyle

7,2ml (51,5 mmoles) de triéthylamine puis 5,9g (15,5 mmoles) du chlorhydrate du chlorure de 4-(2-méthyl-quinolin-4-ylméthoxy)-benzènesulfonyle sont ajoutés à une solution de 3,34g (12,9 mmoles) de dichlorhydrate de (R)-3-amino-2-pipéridin-1-yl-propanoate de méthyle dans 30ml de dichlorométhane et 30ml de diméthylformamide préalablement refroidie par un bain de glace. Le milieu réactionnel est agité à température ambiante pendant 3h30, hydrolysé puis dilué par du dichlorométhane. Le produit est extrait au dichlorométhane. La phase organique est lavée par de l'eau, séchée sur sulfate de magnésium, filtrée et concentrée sous vide. Le résidu obtenu est purifié par chromatographie sur gel de silice élué avec un mélange heptane/acétate d'éthyle 50/50. 4g (62%) de (R)-3-[4-(2-méthyl-quinolin-4-ylméthoxy)-benzènesulfonylamino]-2-pipéridin-1-yl-propanoate de méthyle sont obtenus sous forme d'un solide blanc.

### 16.6 : acide (R)-3-[4-(2-méthyl-quinolin-4-ylméthoxy)-benzènesulfonylamino]-2-pipéridin-1-yl-propanoique

De manière analogue à l'exemple 1.6, à partir de 4,0g (8,0 mmoles) de (R)-3-[4-(2-méthyl-quinolin-4-ylméthoxy)-benzènesulfonylamino]-2-pipéridin-1-yl-propanoate de méthyle, 2,95g (76%) d' acide (R)-3-[4-(2-méthyl-quinolin-4-ylméthoxy)-benzènesulfonylamino]-2-pipéridin-1-yl-propanoïque sont obtenus sous la forme d'un solide blanc.

### 16.7: (R)-N-hydroxy-3-[4-(2-méthyl-quinolin-4-ylméthoxy)-benzènesulfonylamino]-2-pipéridin-1-yl-propionamide

De manière analogue à l'exemple 1.7, à partir de 2,95g (6 mmoles) d'acide (R)-3-[4-(2-méthyl-quinolin-4-ylméthoxy)-benzènesulfonylamino]-2-pipéridin-1-yl-propanoique, 1,8g de résidu brut sont obtenus et recristallisés dans un mélande acétone/eau 50/50. Après filtration, le solide obtenu est à nouveau purifié par chromatographie sur gel de silice élué avec un mélange dichlorométhane/méthanol 95/5. 50mg (2%) de (R)-N-Hydroxy-3-[4-(2-methyl-quinolin-4-ylmethoxy)-benzenesulfonylamino]-2-piperidin-1-yl-propionamide sont obtenus sous la forme d'un solide beige.
RMN ¹H (δ, DMSO) : 1,29-1,31 (m, 2H); 1,39-1,40 (m, 4H); 2,34-2,38 (m, 4H); 2,67 (s, 3H); 2,73-2,79 (m, 1 H); 2,96-2,99 (m, 2H); 5,72 (s, 2H); 7,33-7,37 (m, 3H); 7,57-7,62 (m, 2H); 7,74-7,80 (m, 1 H); 7,98 (d, J=8,4Hz, 2H); 8,11 (d, J=8,4Hz, 2H); 8,86 (s, 1 H), 10,54 (s, 1 H).

### Exemple 17 : Test enzymatique d'inhibition de TACE.

### Description du test

Les produits sont solubilisés dans du DMSO à une concentration de 10 mM. Une dilution sérielle de raison 3 sur 10 points est effectuée de façon à avoir une gamme de concentration allant de 10 µM à 0,5 nM final.
L'enzyme TACE est une production interne (réalisée selon la publication « protein Eng Des Sel 2006, 19., 155-161 ») et, est ajouté de façon à avoir un signal équivalent à 6 fois le bruit de fond en 2h à 37°C. La réaction s'effectue en milieu tamponné Tris 50 mM, 4% de glycérol, pH7,4. Le substrat fluorescent est MCA-Pro-Leu-Ala-Val-(Dpa)-Arg-Ser-Ser-Arg-NH2 (R&D system référence :ES003). Le substrat est clivé par l'enzyme entre l'alanine et la valine libérant ainsi un peptide fluorescent (excitation : 320nm, émission : 420nm). Le substrat est utilisé à 40µM. la réaction est réalisée dans un volume final de 10µl (4µl inhibiteur, 4µl substrat, 2µl enzyme) dans une plaque de 384 puits low volume (Corning référence : 3676). La plaque est incubée 2h à température ambiante, puis lue en fluorescence au Pherastar (BMG labtech). L'IC₅₀ est déterminée en utilisant un logiciel de traitement mathématique (XLfit).

### Test des produits

| n° exemple | % inhibition TACE à 10 µM | IC₅₀ - TACE (nM) |
|---|---|---|
| ex1 | 98 | 181 |
| ex2 | 95 | 298 |
| ex3 | 97 | 181 |
| ex5 | 97 | 249 |
| ex6 | 97 | 328 |
| ex7 | 98 | 147 |
| ex8 | 99 | 26 |
| ex9 | 99 | 469 |
| ex14 | 91 | 38 |
| ex15 | 98 | 112 |

Sur la base des résultats obtenus dans le test enzymatique TACE décrit ci-dessus, les composés de la présente invention sont des inhibiteurs de l'enzyme TNF-alpha converting enzyme (TACE) et de ce fait peuvent être des principes actifs potentiels pour le traitement de pathologies pour lesquelles diminuer la production de TNF alpha serait d'un grand intérêt.

### Exemple 18 : test de sélectivité

### Principe du test :

Les molécules sont testées en dose réponse sur les enzymes suivants MMP1, MMP3, MMP9, ADAM9 et ADAM10 suivant le même protocole que celui décrit pour l'enzyme TACE dans l'exemple 17 mais avec des substrats différents (MMP R&D system référence :P126-990 et ADAM R&D system référence :ES003).
Les enzymes sont achetées chez Calbiochem

### Test des produits :

| | IC50 (nM) | | | | | |
|---|---|---|---|---|---|---|
| Exemple | MMP1 | MMP3 | MMP9 | ADAM9 | ADAM10 | TACE |
| **8** | 3000 | 4500 | >10000 | >10000 | >10000 | 26 |
| **14** | 1983 | 3034 | >10000 | >10000 | >10000 | 24 |
| Apratastat | 145 | 10 | 82 | 85 | 71 | 5 |

Sur la base des résultats obtenus dans le test de sélectivité décrit ci-dessus, ces composés sont également très sélectifs de TACE par rapport aux autres ADAMs et MMPs, c'est-à-dire qu'ils présentent des IC₅₀ pour d'autres ADAMs ou MMPs au moins 10 fois supérieures à celle obtenue pour TACE, et plus avantageusement au moins 100 fois supérieures.
Or, dans la mesure où il est connu que l'inhibition non sélective de ces familles d'enzymes induit des effets secondaires indésirables observés *In Vivo,* l'inhibition sélective de TACE par rapport à ces autres enzymes devrait permettre de réduire des effets secondaires indésirables lors de l'administration de ces molécules pour le traitement de pathologies pour lesquelles diminuer la production de TNF alpha serait d'un grand intérêt.

## Revendications

1. Composé de formule (I) suivante: 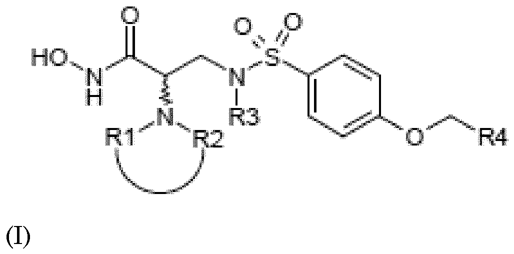 dans laquelle:
R₁ et R₂ forment un cycle avec l'atome d'azote auquel ils sont rattachés,
ledit cycle étant représenté par la formule suivante: 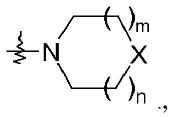
X, m et n ayant les significations données ci-après, R₃ est un atome d'hydrogène ou un radical alkyle en C₁₋₄;
R₄ est un radical alkyle en C₁₋₁₀, un radical alcényle en C₂₋₁₀, un radical alcynyl en C₂₋₁₀, un radical aryle, un radical alkyle en C₁₋₁₀ substitué par un radical aryle qui est non substitué ou substitué, un radical hétérocyclique, un radical cycloalkyle en C₃₋₇, un radical hétéroaryle, ou un radical hétéroaralkyle;
X représente un atome d'oxygène, un radical -CH₂-, un atome de soufre, un radical SO, ou un radical SO₂,
m peut prendre les valeurs de 0 ou 1; et
n peut prendre les valeurs de 0, 1 , 2, ou 3;
ainsi que des sels d'addition dudit composé de formule (I) avec un acide pharmaceutiquement acceptable, des sels d'addition dudit composé de formule (I) avec une base pharmaceutiquement acceptable, et des énantiomères dudit composé de formule (I),
dans laquelle
lesdits radicaux alkyle en C₁₋₁₀, alcényle en C₂₋₁₀, alcynyle en C₂₋₁₀ et cycloalkyle en C₃₋₇ peuvent être substitués par un ou plusieurs radicaux choisis parmi un halogène, un alcoxyle en C₁₋₁₀ et un hydroxyle;
un radical aryle est un cycle hydrocarboné aromatique ou deux cycles condensés hydrocarbonés aromatiques;
un radical hétérocyclique est une chaîne hydrocarbonée cyclique ou polycyclique, saturée ou insaturée, comprenant un ou plusieurs hétéroatomes choisis parmi O, S et N;
lesquels radicaux aryle et hétérocyclique peuvent être substitués par un ou plusieurs groupes choisis parmi un alkyle en C₁₋₁₀, un alcoxyle en C₁₋₁₀, un aryle, un halogène, un hydroxyle, un cyano, un trifluorométhyle et un nitro; un radical hétéroaryle est une chaîne hydrocarbonée cyclique ou polycyclique, aromatique, comprenant un ou plusieurs hétéroatomes choisis parmi O, S et N, lequel radical hétéroaryle peut être substitué par un ou plusieurs groupes choisis parmi un alkyle en C₁₋₁₀, un alcoxyle en C₁₋₁₀, un aryle, un aryle substitué, un halogène, un hydroxy, un cyano, un trifluorométhyle et un nitro;
un radical hétéroaralkyle est un radical alkyle en C₁₋₁₀ substitué par un radical hétéroaryle, lequel radical hétéroaralkyle peut être substitué par un ou plusieurs groupes choisis parmi alkyle en C₁₋₁₀, alcoxyle en C₁₋₁₀, un halogène, un hydroxyle, un cyano, un trifluorométhyle et un nitro;
et
un halogène est un atome de fluor, de chlore, de brome ou d'iode.

2. Les sels d'addition du composé selon revendication 1 avec un acide pharmaceutiquement acceptable, **caractérisés en ce que** l'acide pharmaceutiquement acceptable est choisi dans la liste constituée par l'acide chlorhydrique, l'acide bromhydrique, l'acide sulfurique, l'acide nitrique, l'acide phosphorique, l'acide acétique, l'acide trifluoroacétique, l'acide trichloroacétique, l'acide propionique, l'acide glycolique, l'acide pyruvique, l'acide succinique, l'acide benzoïque, l'acide cinnamique, l'acide mandélique, l'acide méthanesulfonique, l'acide para-toluène sulfonique, l'acide salicylique, l'acide picrique, l'acide citrique, l'acide oxalique, l'acide tartrique, l'acide malonique, l'acide maléique, l'acide camphorsulfonique et l'acide fumarique.

3. Les sels d'addition du composé selon revendication 1 avec une base pharmaceutiquement acceptable, **caractérisés en ce que** la base pharmaceutiquement acceptable est choisie dans la liste constituée par l'hydroxyde de potassium, l'hydroxyde de sodium, l'hydroxyde de lithium, l'hydroxyde de calcium, la méthylamine, l'éthylamine, l'éthanolamine, la propylamine, l'isopropylamine, les 4 isomères de la butylamine, la diméthylamine, la diéthylamine, la diéthanolamine, la dipropylamine, la diisopropylamine, la di-n-butylamine, la pyrrolidine, la pipéridine, la morpholine, la diéthanol phénylamine, la triméthylamine, la triéthylamine la tripropylamine, la quinuclidine, la pyridine, la quinoline, l'isoquinoline, la lysine, l'arginine et l'ornithine.

4. Le composé selon l'une des revendications 1 à 3 **caractérisé en ce que**
R₄ est un radical aryle, un radical alkyle en C₁₋₁₀ substitué par un radical aryle qui est non substitué ou substitué, un radical hétérocyclique, un radical hétéroaryle, ou un radical hétéroaralkyle; et
n peut prendre les valeurs de 0, 1, ou 2;
ainsi que des sels d'addition dudit composé avec un acide pharmaceutiquement acceptable, des sels d'addition dudit composé avec une base pharmaceutiquement acceptable, et des énantiomères dudit composé.

5. Le composé selon l'une des revendications 1 à 3 **caractérisé en ce que**
R₄ est un radical aryle, un radical alkyle en C₁₋₁₀ substitué par un radical aryle qui est non substitué ou substitué, un radical hétérocyclique, un radical hétéroaryle, ou un radical hétéroaralkyle;
X représente un atome d'oxygène, ou un radical -CH₂-;
m prend la valeur 1; et
n peut prendre les valeurs de 0, 1, ou 2;
ainsi que des sels d'addition dudit composé avec un acide pharmaceutiquement acceptable, des sels d'addition dudit composé avec une base pharmaceutiquement acceptable, et des énantiomères dudit composé.

6. Le composé selon l'une des revendications 1 à 3 **caractérisé en ce que**
R₃ est un atome d'hydrogène;
X représente un atome d'oxygène, ou un radical -CH₂-;
m prend la valeur 1; et
n peut prendre les valeurs de 1 ou 2;
ainsi que des sels d'addition dudit composé avec un acide pharmaceutiquement acceptable, des sels d'addition dudit composé avec une base pharmaceutiquement acceptable, et des énantiomères dudit composé.

7. Le composé selon l'une des revendications 1 à 3 **caractérisé en ce que**
R₃ est un atome d'hydrogène;
R₄ est un radical hétérocyclique, un radical hétéroaryle, ou un radical hétéroaralkyle;
X représente un atome d'oxygène ou un radical -CH₂-;
m prend la valeur 1; et
n prend la valeur 1;
ainsi que des sels d'addition dudit composé avec un acide pharmaceutiquement acceptable, des sels d'addition dudit composé avec une base pharmaceutiquement acceptable, et des énantiomères dudit composé.

8. Le composé selon l'une des revendications 1 à 3 **caractérisé en ce que** R₃ est un atome d'hydrogène;
R₄ est un radical hétéroaryle;
X représente un atome d'oxygène ou un radical -CH₂-;
m prend la valeur 1; et
n prend la valeur 1;
ainsi que des sels d'addition dudit composé avec un acide pharmaceutiquement acceptable, des sels d'addition dudit composé avec une base pharmaceutiquement acceptable, et des énantiomères dudit composé.

9. Le composé selon l'une des revendications 1 à 8 **caractérisé en ce que** le composé est choisi dans la liste constituée par les composés suivants:
1) (S)-3-(4-benzyloxy-benzènesulfonylamino)-N-hydroxy-2-pipéridin-1-yl-propionamide
2) (S)-3-(4-benzyloxy-benzènesulfonylamino)-N-hydroxy-2-pyrrolidin-1-yl-propionamide
3) (S)-3-[4-(4-fluoro-benzyloxy)-benzènesulfonylamino]-N-hydroxy-2-pipéridin-1-yl-propionamide
4) (S)-3-[(4-benzyloxy-benzènesulfonyl)-méthyl-amino]-N-hydroxy-2-pipéridin-1-yl-propionamide
5) (S)-N-hydroxy-3-[4-(naphtalen-2-ylméthoxy)-benzènesulfonylamino]-2-pipéridin-1 -yl-propionamide
6) (S)-3-[4-(3,4-dichloro-benzyloxy)-benzènesulfonylamino]-N-hydroxy-2-pipéridin-1 -yl-propionamide
7) (S)-3-(4-benzyloxy-benzènesulfonylamino)-N-hydroxy-2-morpholin-4-yl-propionamide
8) (S)-N-hydroxy-3-[4-(2-méthyl-quinolin-4-ylméthoxy)-benzènesulfonylamino]-2-pipéridin-1-yl-propionamide
9) (S)-3-[4-(3,5-dichloro-benzyloxy)-benzènesulfonylamino]-N-hydroxy-2-pipéridin-1 -yl-propionamide
10) (S)-N-hydroxy-2-pipéridin-1-yl-3-(4-propoxy-benzènesulfonylamino)-propionamide
11) (S)-3-(4-cyclopropylméthoxy-benzènesulfonylamino)-N-hydroxy-2-morpholin-4-yl-propionamide
12) (S)-3-[4-(4-tert-butyl-benzyloxy)-benzènesulfonylamino]-N-hydroxy-2-morpholin-4-yl-propionamide
13) (S)-N-hydroxy-2-morpholin-4-yl-3-(4-phénéthyloxy-benzènesulfonylamino)-propionamide
14) (S)-N-hydroxy-3-[4-(2-méthyl-quinolin-4-ylméthoxy)-benzènesulfonylamino]-2-morpholin-4-yl-propionamide
15) (S)-N-hydroxy-3-[4-(2-méthyl-quinolin-4-ylméthoxy)-benzènesulfonylamino]-2-pipéridin-1-yl-propionamide
16) (R)-N-hydroxy-3- [4-(2-méthyl-quinolin-4-ylméthoxy)-benzènesulfonylamino]-2-pyrrolidin-1-yl-propionamide
17) (R)-N-hydroxy-3-[4-(2-méthyl-quinolin-4-ylméthoxy)-benzènesulfonylamino]-2-pipéridin-1-yl-propionamide
18) (S)-N-hydroxy-3-[4-(2-méthyl-quinolin-4-ylméthoxy)-benzènesulfonylamino]-2-thiomorpholin-4-yl-propionamide
19) (S)-2-(1,1-dioxo-thiomorpholin-4-yl)-N-hydroxy-3-[4-(2-méthyl-quinolin-4-ylméthoxy)-benzènesulfonylamino]-propionamide
20) (S)-2-azepan-1-yl-N-hydroxy-3-[4-(3-méthyl-benzyloxy)-benzènesulfonylamino]-propionamide
21) (S)-2-azepan-1-yl-N-hydroxy-3-[4-(2-méthyl-quinolin-4-ylméthoxy)-benzènesulfonylamino]- propionamide
22) (S)-N-hydroxy-2-pipéridin-1-yl-3-[4-(pyrazolo[1,5-α]pyridin-3-ylméthoxy)-benzènesulfonylamino]-propionamide
23) (S)-N-hydroxy-3-[4-(2-méthyl-pyrazolo[1,5-α]pyridin-3-ylméthoxy)-benzènesulfonylamino]-2- pipéridin-1-yl-propionamide
24) (S)-N-hydroxy-2-pipéridin-1-yl-3-[4-(quinolin-4-ylméthoxy)-benzènesulfonylamino]-propionamide
25) (S)-N-hydroxy-2-morpholin-4-yl-3-[4-(quinolin-4-ylméthoxy)-benzènesulfonylamino]-propionamide
26) (S)-N-hydroxy-3-{propyl-[4-(quinolin-4-ylméthoxy)-benzènesulfonyl]-amino}-2-pyrrolidin-1-yl-propionamide
27) (S)-N-hydroxy-3-[4-(3-méthyl-benzyloxy)-benzènesulfonylamino]-2-[1,4]oxazocan-4-yl-propionamide
28) (S)-2-azocan-1-yl-N-hydroxy-3-[4-(pyrimidin-4-ylméthoxy)-benzènesulfonylamino]-propionamide
29) (S)-N-hydroxy-2-morpholin-4-yl-3-[4-(pyrazolo[1,5-α]pyridin-3-ylméthoxy)-benzènesulfonylamino]-propionamide
30) (S)-N-hydroxy-3-[4-(pyrazolo[1,5-α]pyridin-3-ylméthoxy)-benzènesulfonylamino]-2-thiomorpholin-4-yl-propionamide
31) (S)-N-hydroxy-2-pipéridin-1-yl-3-[4-(4-propoxy-benzyloxy)-benzènesulfonylamino]-propionamide
32) (R)-N-hydroxy-3- [4-(2-méthyl-quinolin-4-ylméthoxy)-benzènesulfonylamino]-2-morpholin-4-yl-propionamide.
33) (S)-N-hydroxy-3-[4-(2-méthyl-1H-indol-3-ylméthoxy)-benzènesulfonylamino]-2-pipéridin-1-yl-propionamide
34) (S)-N-hydroxy-3-[4-(2-isopropyl-benzofuran-3-ylméthoxy)-benzènesulfonylamino]-2-morpholin-4-yl-propionamide et
35) (S)-2-azétidin-1-yl-N-hydroxy-3-[4-(2-méthyl-quinolin-4-ylméthoxy)-benzènesulfonylamino]-propionamide.

10. Le composé, les sels d'addition dudit composé avec un acide pharmaceutiquement acceptable, les sels d'addition dudit composé avec une base pharmaceutiquement acceptable, ou les énantiomères dudit composé selon l'une des revendications 1 à 9 en tant que médicament.

11. Le composé selon l'une des revendications 1 à 9 pour son utilisation dans le traitement de maladies ou désordres inflammatoires impliquant une production de TNFα, choisis parmi le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'attaque ischémique, le rejet de greffe, le cancer, l'athérosclérose, l'obésité, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète mellitus non insulino dépendant, les maladies allergiques, l'asthme, la maladie d'obstruction pulmonaire chronique (COPD) et l'inflammation oculaire.

12. Le composé selon l'une des revendications 1 à 9 pour son utilisation dans le traitement des maladies inflammatoires de la peau, du psoriasis, de la dermatite atopique et du rhumatisme psoriasique.

13. Le composé selon l'une des revendications 1 à 9 pour son utilisation dans le traitement des pathologies neurologiques à caractère inflammatoire impliquant une production de TNFα, choisies parmi la maladie d'Alzheimer, la maladie de Parkinson, les désordres parkinsoniens, la sclérose amyotrophique latérale, les maladies auto-immunes du système nerveux, les maladies autonomiques du système nerveux, les douleurs dorsales, l'oedème cérébral, les désordres cérébrovasculaires, la démence, les maladies auto-immunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barré, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux central, les maladies d'Huntington et l'attaque cérébrale.

14. Composition pharmaceutique **caractérisée en ce qu'**elle comprend, dans un support pharmaceutiquement acceptable et compatible avec le mode d'administration retenu pour cette composition, au moins un composé selon l'une des revendications 1 à 9, ledit composé pouvant également se présenter sous une de ses formes énantiomériques ou sous la forme d'un de ses sels pharmaceutiquement acceptables.

15. Le composé selon revendication 1, **caractérisé en ce que** le composé est: (S)-N-hydroxy-3-[4-(2-méthyl-quinolin-4-ylméthoxy)-benzènesulfonylamino]-2-pipéridin-1-yl-propionamide ou (S)-N-hydroxy-3-[4-(2-méthyl-quinolin-4-ylméthoxy)-benzènesulfonylamino]-2-morpholin-4-yl-propionamide.

## Patentansprüche

1. Verbindung der untenstehenden Formel (1): 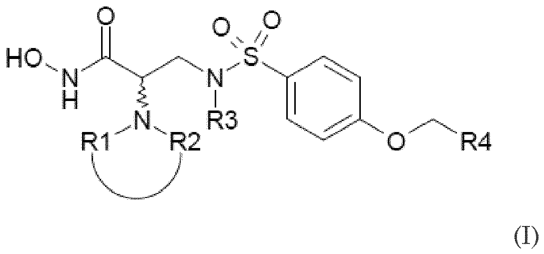 wobei:
R₁ und R₂ einen Ring mit dem Stickstoffatom bilden, an das sie gebunden sind, wobei der Ring durch die untenstehende Formel dargestellt wird: 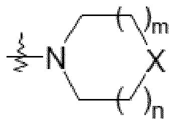
wobei X, m und n die im Folgenden angegebenen Bedeutungen aufweisen;
R₃ ein Wasserstoffatom oder ein C₁₋₄ Alkylradikal ist;
R₄ ein C₁₋₁₀ Alkylradikal, ein C₂₋₁₀ Alkenylradikal, ein C₂₋₁₀ Alkinylradikal, ein Arylradikal, ein C₁₋₁₀ Alkylradikal substitutiert mit einem Arylradikal, das unsubstituiert oder substituiert ist, ein heterocyklisches Radikal, ein C₃₋₇ Cycloalkylradikal, ein Heteroarylradikal, oder ein Heteroaralkylradikal ist;
X ein Sauerstoffatom, ein -CH₂- Radikal, ein Schwefelatom, ein SO Radikal,
oder ein SO₂ Radikal darstellt;
m die Werte von 0 oder 1 annehmen kann; und
n die Werte von 0, 1, 2, oder 3 annehmen kann;
und ferner die Additionssalze der Verbindung der Formel (I) mit einer pharmazeutisch akzeptablen Säure, Additionssalze der Verbindung der Formel (I) mit einer pharmazeutisch akzeptablen Base, und Enantiomere der Verbindung der Formel (I), wobei
die C₁₋₁₀ Alkyl, C₂₋₁₀ Alkenyl, C₂₋₁₀ Alkinyl und C₃₋₇ Cycloalkyl-Radikale mit einem oder mehreren Radikalen ausgewählt aus einem Halogen, einem C₁₋₁₀ Alkoxy und einem Hydroxyl substituiert sein können;
ein Arylradikal ein aromatischer kohlenwasserstoff-basierter Ring oder zwei kondensierte aromatische kohlenwasserstoff-basierte Ringe ist;
ein heterocyclisches Radikal eine gesättigte oder ungesättigte cyclische oder polycyclische kohlenwasserstoff-basierte Kette ist, die ein oder mehrere Heteroatome ausgewählt aus O, S und N umfasst;
welche Aryl- und heterocyclischen Radikale substituiert sein können mit einer oder mehreren Gruppen ausgewählt aus einem C₁₋₁₀ Alkyl, einem C₁₋₁₀ Alkoxy, einem Aryl, einem Halogen, einem Hydroxyl, einem Cyano, einem Trifluormethyl und einer Nitrogruppe;
ein Heteroarylradikal eine cyclische oder polycyclische aromatische kohlenwasserstoff-basierte Kette ist, die ein oder mehrere Heteroatome ausgewählt aus O, S und N umfasst, welches Heteroarylradikal mit einer oder mehreren Gruppen ausgewählt aus einem C₁₋₁₀ Alkyl, einem C₁₋₁₀ Alkoxy, einem Aryl, einem substituierten Aryl, einem Halogen, einem Hydroxyl, einem Cyano, einem Trifluormethyl und einer Nitrogruppe substituiert sein können;
ein Heteroaralkylradikal ein C₁₋₁₀ Alkylradikal ist, das mit einem Heteroarylradikal substituiert ist,
welches Heteroaralkylradikal mit einer oder mehreren Gruppen ausgewählt aus einem C₁₋₁₀ Alkyl, einem C₁₋₁₀ Alkoxy, einem Halogen, einem Hydroxyl, einem Cyano, einem Trifluormethyl und einer Nitrogruppe substituiert sein kann;
und ein Halogen ein Fluor-, Chlor-, Brom-oder Iodatom ist.

2. Additionssalze der Verbindung wie in Anspruch 1 beansprucht mit einer pharmazeutisch akzeptablen Säure, **dadurch gekennzeichnet, dass** die pharmazeutisch akzeptable Säure ausgewählt ist aus der Gruppe bestehend aus Salzsäure, Bromwasserstoffsäure, Schwefelsäure, Salpetersäure Phosphorsäure, Essigsäure, Trifluoressigsäure, Trichloressigsäure, Propionsäure, Glycolsäure, Brenztraubensäure, Bernsteinsäure, Benzoesäure, Zimtsäure , Mandelsäure, Methansulfonsäure, para-Toluolsulfonsäure, Salicylsäure, Pikrinsäure, Zitronensäure, Oxalsäure, Weinsäure, Malonsäure, Maleinsäure, Camphersulfonsäure und Fumarsäure.

3. Additionssalze der Verbindung wie in Anspruch 1 beansprucht mit einer pharmazeutisch akzeptablen Base, **dadurch gekennzeichnet, dass** die pharmazeutisch akzeptable Base ausgewählt ist aus der Gruppe bestehend aus Kaliumhydroxid, Natriumhydroxid, Lithiumhydroxid, Calciumhydroxid, Methylamin, Ethylamin, Ethanolamin, Propylamin, Isopropylamin, den vier Isomeren von Butylamin, Dimethylamin, Diethylamin, Diethanolamin, Dipropylamin, Diisopropylamin, Di-n-butylamin, Pyrrolidin, Piperidin, Morpholin, Diethanolphenylamin, Trimethylamin, Triethylamin, Tripropylamin, Chinuclidin, Pyridin, Chinolin, Isochinolin, Lysin, Arginin und
Ornithin.

4. Verbindung wie in einem der Ansprüche 1 bis 3 beansprucht, **dadurch gekennzeichnet, daß**
R₄ ein Arylradikal, ein C₁₋₁₀ Alkylradikal, das mit einem unsubstituierten oder substituierten Arylradikal substituiert ist, ein heterocyclisches Radikal, ein Heteroarylradikal oder ein Heteroaralkylradikal ist; und
n die Werte von 0, 1, oder 2 annehmen kann;
und auch Additionssalze der Verbindung mit einer pharmazeutisch akzeptablen Säure, Additionssalze der Verbindung mit einer pharmazeutisch akzeptablen Base, und Enantiomere der Verbindung.

5. Verbindung wie in einem der Ansprüche 1 bis 3 beansprucht, **dadurch gekennzeichnet, daß**
R₄ ein Arylradikal, ein C₁₋₁₀ Alkylradikal, das mit einem unsubstituierten oder substituierten Arylradikal substituiert ist, ein heterocyclisches Radikal, ein Heteroarylradikal oder ein Heteroaralkylradikal ist,
X ein Sauerstoffatom oder ein -CH₂- Radikal darstellt;
m den Wert von 1 annimmt; und
n die Werte von 0, 1, oder 2 annehmen kann;
und auch Additionssalze der Verbindung mit einer pharmazeutisch akzeptablen Säure, Additionssalze der Verbindung mit einer pharmazeutisch akzeptablen Base, und Enantiomere der Verbindung.

6. Verbindung wie in einem der Ansprüche 1 bis 3 beansprucht, **dadurch gekennzeichnet, daß**
R₃ ein Wasserstoffatom ist;
X ein Sauerstoffatom oder ein -CH₂- Radikal darstellt;
m den Wert von 1 annimmt; und
n die Werte von 1 oder 2 annehmen kann;
und auch Additionssalze der Verbindung mit einer pharmazeutisch akzeptablen Säure, Additionssalze der Verbindung mit einer pharmazeutisch akzeptablen Base, und Enantiomere der Verbindung.

7. Verbindung wie in einem der Ansprüche 1 bis 3 beansprucht, **dadurch gekennzeichnet, daß**
R₃ ein Wasserstoffatom ist;
R₄ ein heterocyclisches Radikal, ein Heteroarylradikal oder ein Heteroaralkylradikal ist;
X ein Sauerstoffatom oder ein -CH₂- Radikal darstellt;
m den Wert von 1 annimmt; und
n den Wert von 1 annimmt;
und auch Additionssalze der Verbindung mit einer pharmazeutisch akzeptablen Säure, Additionssalze der Verbindung mit einer pharmazeutisch akzeptablen Base, und Enantiomere der Verbindung.

8. Verbindung wie in einem der Ansprüche 1 bis 3 beansprucht, **dadurch gekennzeichnet, daß**
R₃ ein Wasserstoffatom ist;
R₄ ein Heteroarylradikal ist;
X ein Sauerstoffatom oder ein -CH₂- Radikal darstellt;
m den Wert von 1 annimmt; und
n den Wert von 1 annimmt;
und auch Additionssalze der Verbindung mit einer pharmazeutisch akzeptablen Säure, Additionssalze der Verbindung mit einer pharmazeutisch akzeptablen Base, und Enantiomere der Verbindung.

9. Verbindung wie in einem der Ansprüche 1 bis 8 beansprucht, **dadurch gekennzeichnet, daß** die Verbindung ausgewählt ist aus der Gruppe bestehend aus:
1) (S)-3-(4-Benzyloxy-benzolsulfonylamino)-N-hydroxy-2-piperidin-1-yl-propionamid
2) (S)-3-(4-Benzyloxy-benzolsulfonylamino)-N-hydroxy-2-pyrrolidin-1-yl-propionamid
3) (S)-3-[4-(4-Fluor-benzyloxy)-benzolsulfonylamino]-N-hydroxy-2-piperidin-1-yl-propjonamide
4) (S)-3-[(4-Benzyloxy-benzolsulfonyl)-methyl-amino]-N-hydroxy-2-piperidin-1-yl- propionamid
5) (S)-N-Hydroxy-3-[4-(Naphthalin-2-ylmethoxy)-benzolsulfonylamino]-2-piperidin-1-yl-propionamid
6) (S)-3-[4-(3,4-Dichlor-benzyloxy)-benzolsulfonylamino]-N-hydroxy-2-piperidin-1-yl-propionamid
7) (S)-3-(4-Benzyloxy-benzolsulfonylamino)-N-hydroxy-2-morpholin-4-yl propionamid
8) (S)-N-Hydroxy-3-[4-(2-methyl-chinolin-4-ylmethoxy)-benzolsulfonylamino] -2-piperidin-1-yl-propionamid
9) (S)-3-[4-(3,5-dichlor-benzyloxy)-benzolsulfonylamino]-N-Hydroxy-2-piperidin-1 -yl-propionamid
10) (S)-N-Hydroxy-2-piperidin-1-yl-3-(4-propoxy-benzolsulfonylamino) propionamid
11) (S)-3-(4-cyclopropylmethoxy-benzolsulfonylamino)-N-hydroxy-2-morpholin-4-yl-propionamid
12) (S)-3-[4-(4-tert-Butyl-benzyloxy)-benzolsulfonylamino]-N-hydroxy-2-morpholin-4-yl-propionamid
13) (S)-N-Hydroxy-2-morpholin-4-yl-3-(4-Phenethyloxy-benzolsulfonylamino) propionamid
14) (S)-N-Hydroxy-3-[4-(2-methyl-chinolin-4-ylmethoxy)-benzolsulfonylamino]-2-morpholin-4-yl-propionamid
15) (S)-N-Hydroxy-3-[4-(2-methyl-chinolin-4-ylmethoxy)-benzolsulfonylamino]-2-piperidin-1-yl-propionamid
16) (R)-N-Hydroxy-3-[4-(2-methyl-chinolin-4-ylmethoxy)-benzolsulfonylamino]-2-pyrrolidin-1-yl-propionamid
17) (R)-N-Hydroxy-3-[4-(2-methyl-chinolin-4-ylmethoxy)-benzolsulfonylamino]-2-piperidin-1-yl-propionamid
18) (S)-N-Hydroxy-3-[4-(2-methyl-chinolin-4-ylmethoxy)-benzolsulfonylamino] -2-thiomorpholin-4-ylpropionamide
19) (S)-2-(1,1-dioxo-thiomorpholin-4-yl)-N-hydroxy-3-[4-(2-methyl-chinolin-4-ylmethoxy)-benzenesulfonylamino-propionamid
20) (S)-2-azepan-1-yl-N-hydroxy-3-[4-(3-methyl-benzyloxy)-benzolsulfonylamino] -propionamid
21) (S)-2-azepan-1-yl-N-hydroxy-3-[4-(2-methyl-chinolin-4-ylmethoxy)-benzolsulfonylamino]-propionamid
22) (S)-N-Hydroxy-2-piperidin-1-yl-3-[4-(pyrazol[1,5-a]pyridin-3-ylmethoxy) -benzolsulfonylamino]-propionamid
23) (S)-N-Hydroxy-3-[4-(2-methyl-pyrazolo[1,5-a]pyridin-3-ylmethoxy)-benzolsulfonylamino]-2-piperidin-1-yl-propionamid
24) (S)-N-Hydroxy-2-piperidin-1-yl-3-[4-(chinolin-4-ylmethoxy)-benzolsulfonylamino]-propionamid
25) (S)-N-Hydroxy-2-morpholin-4-yl-3-[4-(chinolin-4-ylmethoxy)-benzolsulfonylamino]-propionamid
26) (S)-N-Hydroxy-3-propyl-[4-(Chinolin-4-ylmethoxy)-benzolsulfonyl]-amino}-2-pyrrolidin-1-yl-propionamid
27) (S)-N-Hydroxy-3-[4-(3-methyl-benzyloxy)-benzolsulfonylamino]-2-[1,4] oxazocan-4-yl-propionamid
28) (S)-2-azocan-1-yl-N-hydroxy-3-[4-(Pyrimidin-4-ylmethoxy)-benzolsulfonylamino]-propionamid
29) (S)-N-Hydroxy-2-morpholin-4-yl-3-[4-(pyrazol[1,5-a]pyridin-3-ylmethoxy) -benzolsulfonylamino]-propionamid
30) (S)-N-Hydroxy-3-[4-(pyrazol[1,5-a]pyridin-3-ylmethoxy)-benzolsulfonylamino]-2-thiomorpholin-4-yl-propionamid
31) (S)-N-Hydroxy-2-piperidin-1-yl-3-[4-(4-propoxy-benzyloxy)-benzolsulfonylamino]-propionamid
32) (R)-N-Hydroxy-3-[4-(2-methyl-chinolin-4-ylmethoxy)-benzolsulfonylamino]-2-morpholin-4-yl-propionamid.
33) (S)-N-Hydroxy-3-[4-(2-methyl-1H-indol-3-ylmethoxy)-benzolsulfonylamino]-2-piperidin-1-yl-propionamid
34) (S)-N-Hydroxy-3-[4-(2-isopropyl-benzofuran-3-ylmethoxy)-benzolsulfonylamino]-2-morpholin-4-ylpropionamide und
35) (S)-2-azetidin-1-yl-N-hydroxy-3-[4-(2-methyl-chinolin-4-ylmethoxy)-benzensulfonylamino]-propionamid.

10. Verbindung, Additionssalze der Verbindung mit einer pharmazeutisch akzeptablen Säure, Additionssalze der Verbindung mit einer pharmazeutisch akzeptablen Base, oder Enantiomere der Verbindung wie in einem der Ansprüche 1 bis 9 beansprucht, als ein Medikament.

11. Verbindung wie in einem der Ansprüche 1 bis 9 beansprucht zur Verwendung in der Behandlung von entzündlichen Erkrankungen oder Störungen, die mit TNF alpha Produktion einhergehen, ausgewählt aus der Gruppe bestehend aus septischem Schock, hämodynamischem Schock, Malaria, entzündlicher Darmerkrankung (IBD) wie etwa Morbus Crohn und Colitis ulcerosa, entzündlichen Knochenerkrankungen, mykobakteriellen Infektionen, Meningitis, fibrotischen Erkrankungen, Herzerkrankungen, Schlaganfall, Transplantatabstoßung, Krebs, Arteriosklerose, Fettsucht, Erkrankungen, die mit Angiogeneseerscheinungen einhergehen, Autoimmunerkrankungen, Ostoarthritis, rheumatoider Arthritis, Morbus Bechterew, juveniler rheumatoider Arthritis, Multipler Sklerose, HIV, insulinunabhängigem Diabetes Mellitus, allergischen Krankheiten, Asthma, chronisch-obstruktiver Lungenerkrankung (COPD) und Augenentzündung.

12. Verbindung wie in einem der Ansprüche 1 bis 9 beansprucht zur Verwendung in der Behandlung von entzündlichen Erkrankungen der Haut, Psoriasis, atopischer Dermatitis und Psoriasisarthritis.

13. Verbindung wie in einem der Ansprüche 1 bis 9 beansprucht zur Verwendung in der Behandlung von neurologischen pathologischen Zuständen von entzündlicher Art, die mit TNF alpha Produktion einhergehen, ausgewählt aus der Gruppe bestehend aus Alzheimer-Krankheit, Parkinson-Krankheit, parkinsonartige Störungen, amyotropher lateraler Sklerose, Autoimmunkrankheiten des Nervensystems, autonomen Erkrankungen des Nervensystems, Kreuzschmerzen, Hirnödem, cerebrovaskulären Störungen, Demenz, Autoimmunerkrankungen der Demyelinisierung der Nervenfasern des Nervensystems, diabetischer Neuropathie, Enzephalitis, Enzephalomyelitis, Epilepsie, chronische Müdigkeit, Riesenzellarteriitis, Guillain-Barré-Syndrom, Kopfschmerzen, Multipler Sklerose, Nervenschmerzen, Erkrankungen des peripheren Nervensystems, Polyneuropathien, Polyradiculoneuropathie, Radikulopathie, Atemlähmung, Rückenmark-Krankheit, Tourette Syndrom, Vaskulitis des Zentralnervensystems, Huntington Krankheit und Schlaganfall.

14. Pharmazeutische Zusammensetzung, **dadurch gekennzeichnet, dass** sie in einem Träger, der pharmazeutisch akzeptabel und kompatibel mit der Art der für diese Zusammensetzung ausgewählten Verabreichung ist, mindestens eine Verbindung wie in einem der Ansprüche 1 bis 9 beansprucht enthält, wobei die Verbindung auch in einer ihrer enantiomeren Formen oder in der Form eines ihrer pharmazeutisch akzeptablen Salze vorliegen kann.

15. Verbindung wie in Anspruch 1 beansprucht, wobei die Verbindung ist:
(S)-N-Hydroxy-3-[4-(2-methylchinolin-4-ylmethoxy)benzensulfonylamino]-2-piperidin-1-yl-propionamid oder
(S)-N-Hydroxy-3-[4-(2-methylchinolin-4-ylmethoxy)benzensulfonylamino]-2-morpholin-4-yl-propionamid.

## Claims

1. A compound of formula (I) below: 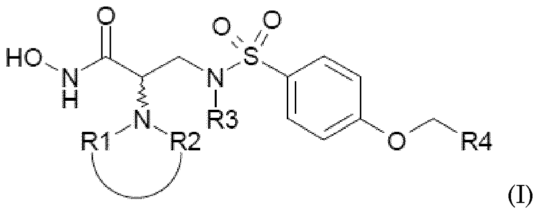 in which:
R₁ and R₂ form a ring with the nitrogen atom to which they are attached, said ring being represented by the formula below: 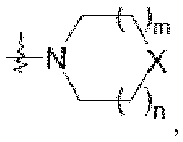
X, m and n having the meanings given hereinafter;
R₃ is a hydrogen atom or a C₁₋₄ alkyl radical;
R₄ is a C₁₋₁₀ alkyl radical, a C₂₋₁₀ alkenyl radical, a C₂₋₁₀ alkynyl radical, an aryl radical, a C₁₋₁₀ alkyl radical substituted with an aryl radical that is unsubstituted or substituted, a heterocyclic radical, a C₃₋₇ cycloalkyl radical, a heteroaryl radical, or a heteroaralkyl radical;
X represents an oxygen atom, a -CH₂- radical, a sulfur atom, an SO radical, or
an SO₂ radical;
m can take the values of 0 or 1; and
n can take the values of 0, 1, 2, or 3;
and also addition salts of said compound of formula (I) with a pharmaceutically acceptable acid, addition salts of said compound of formula (I) with a pharmaceutically acceptable base, and enantiomers of said compound of formula (I), wherein
said C₁₋₁₀ alkyl, C₂₋₁₀ alkenyl, C₂₋₁₀ alkynyl and C₃₋₇ cycloalkyl radicals may be substituted with one or more radicals selected from a halogen, a C₁₋₁₀ alkoxy and a hydroxyl;
an aryl radical is an aromatic hydrocarbon-based ring or two condensed aromatic hydrocarbon-based rings;
a heterocyclic radical is a saturated or unsaturated, cyclic or polycyclic hydrocarbon-based chain comprising one or more heteroatoms selected from O, S and N;
which aryl and heterocyclic radicals may be substituted with one or more groups selected from a C₁₋₁₀ alkyl, a C₁₋₁₀ alkoxy, an aryl, a halogen, a hydroxyl, a cyano, a trifluoromethyl and a nitro;
a heteroaryl radical is a cyclic or polycyclic aromatic hydrocarbon-based chain comprising one or more heteroatoms selected from O, S and N, which heteroaryl radical may be substituted with one or more groups selected from a C₁₋₁₀ alkyl, a C₁₋₁₀ alkoxy, an aryl, a substituted aryl, a halogen, a hydroxyl, a cyano, a trifluoromethyl and a nitro;
a heteroaralkyl radical is a C₁₋₁₀ alkyl radical substituted with a heteroaryl radical,
which heteroaralkyl radical may be substituted with one or more groups selected from C₁₋₁₀ alkyl, C₁₋₁₀ alkoxy, a halogen, a hydroxyl, a cyano, a trifluoromethyl and a nitro; and a halogen is a fluorine, chlorine, bromine or iodine atom.

2. The addition salts of the compound as claimed in claim 1, with a pharmaceutically acceptable acid, **characterized in that** the pharmaceutically acceptable acid is selected from the group consisting of hydrochloric acid, hydrobromic acid, sulfuric acid, nitric acid, phosphoric acid, acetic acid, trifluoroacetic acid, trichloroacetic acid, propionic acid, glycolic acid, pyruvic acid, succinic acid, benzoic acid, cinnamic acid, mandelic acid, methanesulfonic acid, para-toluenesulfonic acid, salicylic acid, picric acid, citric acid, oxalic acid, tartaric acid, malonic acid, maleic acid, camphorsulfonic acid, and fumaric acid.

3. The addition salts of the compound as claimed in claim 1, with a pharmaceutically acceptable base, **characterized in that** the pharmaceutically acceptable base is selected from the group consisting of potassium hydroxide, sodium hydroxide, lithium hydroxide, calcium hydroxide, methylamine, ethylamine, ethanolamine, propylamine, isopropylamine, the 4 isomers of butylamine, dimethylamine, diethylamine, diethanolamine, dipropylamine, diisopropylamine, di-n-butylamine, pyrrolidine, piperidine, morpholine, diethanolphenylamine, trimethylamine, triethylamine, tripropylamine, quinuclidine, pyridine, quinoline, isoquinoline, lysine, arginine, and ornithine.

4. The compound as claimed in any one of claims 1 to 3, **characterized in that**
R₄ is an aryl radical, a C₁₋₁₀ alkyl radical substituted with an aryl radical that is unsubstituted or substituted, a heterocyclic radical, a heteroaryl radical, or a heteroaralkyl radical; and
n can take the values of 0, 1, or 2;
and also addition salts of said compound with a pharmaceutically acceptable acid, addition salts of said compound with a pharmaceutically acceptable base, and enantiomers of said compound.

5. The compound as claimed in any one of claims 1 to 3, **characterized in that**
R₄ is an aryl radical, a C₁₋₁₀ alkyl radical substituted with an aryl radical that is unsubstituted or substituted, a heterocyclic radical, a heteroaryl radical, or a heteroaralkyl radical;
X represents an oxygen atom or a -CH₂- radical;
m takes the value of 1; and
n can take the values of 0, 1 or 2;
and also addition salts of said compound with a pharmaceutically acceptable acid, addition salts of said compound with a pharmaceutically acceptable base, and enantiomers of said compound.

6. The compound as claimed in any one of claims 1 to 3, **characterized in that**
R₃ is a hydrogen atom;
X represents an oxygen atom or a -CH₂- radical;
m takes the value of 1; and
n can take the values of 1 or 2;
and also addition salts of said compound with a pharmaceutically acceptable acid, addition salts of said compound with a pharmaceutically acceptable base, and enantiomers of said compound.

7. The compound as claimed in any one of claims 1 to 3, **characterized in that**
R₃ is a hydrogen atom;
R₄ is a heterocyclic radical, a heteroaryl radical, or a heteroaralkyl radical;
X represents an oxygen atom or a -CH₂- radical;
m takes the value of 1; and
n takes the value of 1;
and also addition salts of said compound with a pharmaceutically acceptable acid, addition salts of said compound with a pharmaceutically acceptable base, and enantiomers of said compound.

8. The compound as claimed in any one of claims 1 to 3, **characterized in that**
R₃ is a hydrogen atom;
R₄ is a heteroaryl radical;
X represents an oxygen atom or a -CH2- radical;
m takes the value of 1; and
n takes the value of 1;
and also addition salts of said compound with a pharmaceutically acceptable acid, addition salts of said compound with a pharmaceutically acceptable base, and enantiomers of said compound.

9. The compound as claimed in any one of claims 1 to 8, **characterized in that** the compound is selected from the group consisting of:
1) (S)-3-(4-benzyloxybenzenesulfonylamino)-N-hydroxy-2-piperidin-1-ylpropionamide
2) (S)-3-(4-benzyloxybenzenesulfonylamino)-N-hydroxy-2-pyrrolidin-1-ylpropionamide
3) (S)-3-[4-(4-fluorobenzyloxy)benzenesulfonylamino]-N-hydroxy-2-piperidin-1-ylpropionamide
4) (S)-3-[(4-benzyloxybenzenesulfonyl)methylamino]-N-hydroxy-2-piperidin-1-ylpropionamide
5) (S)-N-hydroxy-3-[4-(naphthalen-2-ylmethoxy)benzenesulfonylamino]-2-piperidin-1-ylpropionamide
6) (S)-3-[4-(3,4-dichlorobenzyloxy)benzenesulfonylamino]-N-hydroxy-2-piperidin-1-ylpropionamide
7) (S)-3-(4-benzyloxybenzenesulfonylamino)-N-hydroxy-2-morpholin-4-ylpropionamide
8) (S)-N-hydroxy-3-[4-(2-methylquinolin-4-ylmethoxy)benzenesulfonylamino]-2-piperidin-1-ylpropionamide
9) (S)-3-[4-(3,5-dichlorobenzyloxy)benzenesulfonylamino]-N-hydroxy-2-piperidin-1-ylpropionamide
10) (S)-N-hydroxy-2-piperidin-1-yl-3-(4-propoxybenzenesulfonylamino)propionamide
11) (S)-3-(4-cyclopropylmethoxybenzenesulfonylamino)-N-hydroxy-2-morpholin-4-ylpropionamide
12) (S)-3-[4-(4-tert-butylbenzyloxy)benzenesulfonylamino]-N-hydroxy-2-morpholin-4-ylpropionamide
13) (S)-N-hydroxy-2-morpholin-4-yl-3-(4-phenethyloxybenzenesulfonylamino)propionamide
14) (S)-N-hydroxy-3-[4-(2-methylquinolin-4-ylmethoxy)benzenesulfonylamino]-2-morpholin-4-ylpropionamide
15) (S)-N-hydroxy-3-[4-(2-methylquinolin-4-ylmethoxy)benzenesulfonylamino]-2-piperidin-1-ylpropionamide
16) (R)-N-hydroxy-3-[4-(2-methylquinolin-4-ylmethoxy)benzenesulfonylamino]-2-pyrrolidin-1-ylpropionamide
17) (R)-N-hydroxy-3-[4-(2-methylquinolin-4-ylmethoxy)benzenesulfonylamino]-2-piperidin-1-yl-propionamide
18) (S)-N-hydroxy-3-[4-(2-methylquinolin-4-ylmethoxy)benzenesulfonylamino]-2-thiomorpholin-4-yl-propionamide
19) (S)-2-(1,1-dioxothiomorpholin-4-yl)-N-hydroxy-3-[4-(2-methylquinolin-4-ylmethoxy)benzenesulfonylamino]propionamide
20) (S)-2-azepan-1-yl-N-hydroxy-3-[4-(3-methylbenzyloxy)benzenesulfonylamino] propionamide
21) (S)-2-azepan-1-yl-N-hydroxy-3-[4-(2-methylquinolin-4-ylmethoxy)benzenesulfonylamino]-propionamide
22) (S)-N-hydroxy-2-piperidin-1-yl-3-[4-(pyrazolo[1,5-a]pyridin-3-ylmethoxy)benzenesulfonylamino]-propionamide
23) (S)-N-hydroxy-3-[4-(2-methylpyrazolo[1,5-a]pyridin-3-ylmethoxy)benzenesulfonylamino]-2-piperidin-1-ylpropionamide
24) (S)-N-hydroxy-2-piperidin-1-yl-3-[4-(quinolin-4-ylmethoxy)benzenesulfonylamino]propionamide
25) (S)-N-hydroxy-2-morpholin-4-yl-3-[4-(quinolin-4-ylmethoxy)benzenesulfonylamino]propionamide
26) (S)-N-hydroxy-3-{propyl[4-(quinolin-4-ylmethoxy)benzenesulfonyl]amino}-2-pyrrolidin-1-yl-propionamide
27) (S)-N-hydroxy-3-[4-(3-methylbenzyloxy)benzenesulfonylamino]-2-[1,4]-oxazocan-4-yl-propionamide
28) (S)-2-azocan-1-yl-N-hydroxy-3-[4-(pyrimidin-4-ylmethoxy)benzenesulfonylamino]propionamide
29) (S)-N-hydroxy-2-morpholin-4-yl-3-[4-(pyrazolo[1,5-a]pyridin-3-ylmethoxy)benzenesulfonylamino]-propionamide
30) (S)-N-hydroxy-3-[4-(pyrazolo[1,5-a]pyridin-3-ylmethoxy) benzenesulfonylamino]-2-thiomorpholin-4-ylpropionamide
31) (S)-N-hydroxy-2-piperidin-1-yl-3-[4-(4-propoxybenzyloxy)benzenesulfonylamino]propionamide
32) (R)-N-hydroxy-3-[4-(2-methylquinolin-4-ylmethoxy)benzenesulfonylamino]-2-morpholin-4-yl-propionamide
33)(S)-N-hydroxy-3-[4-(2-methyl-1H-indol-3-ylmethoxy)benzenesulfonylamino]-2-piperidin-1-yl-propionamide
34)(S)-N-hydroxy-3-[4-(2-isopropylbenzofuran-3-ylmethoxy)benzenesulfonylamino]-2-morpholin-4-yl-propionamide and
35)(S)-2-azetidin-1-yl-N-hydroxy-3-[4-(2-methylquinolin-4-ylmethoxy) benzenesulfonylamino]-propionamide.

10. The compound, addition salts of said compound with a pharmaceutically acceptable acid, addition salts of said compound with a pharmaceutically acceptable base, or enantiomers of said compound as claimed in any one of claims 1 to 9, as a medicament.

11. The compound as claimed in any one of claims 1 to 9, for use in the treatment of
inflammatory diseases or disorders involving TNFα production, selected from the group consisting of septic shock, hemodynamic shock, malaria, inflammatory bowel disease (IBDs) such as Crohn's disease and ulcerative colitis, inflammatory bone diseases, mycobacterial infections, meningitis, fibrotic diseases, cardiac diseases, ischemic attack, transplant rejection, cancer, atherosclerosis, obesity, diseases involving angiogenesis phenomena, autoimmune diseases, osteoarthritis, rheumatoid arthritis, ankylosing spondylitis, juvenile chronic arthritis, multiple sclerosis, HIV, non-insulindependent diabetes mellitus, allergic diseases, asthma, chronic obstructive pulmonary disease (COPD) and ocular inflammation.

12. The compound as claimed in any one of claims 1 to 9, for use in the treatment of inflammatory skin diseases, psoriasis, atopic dermatitis and psoriatic arthritis.

13. The compound as claimed in any one of claims 1 to 9, for use in the treatment of neurological pathological conditions with an inflammatory nature involving TNFα production, selected from the group consisting of Alzheimer's disease, Parkinson's disease, parkinsonian disorders, amyotrophic lateral sclerosis, autoimmune diseases of the nervous system, autonomic diseases of the nervous system, dorsal pain, cerebral edema, cerebrovascular disorders, dementia, nervous system nerve fiber demyelinating autoimmune diseases, diabetic neuropathies, encephalitis, encephalomyelitis, epilepsy, chronic fatigue syndrome, giant cell arteritis, Guillain-Barre syndrome, headaches, multiple sclerosis, neuralgia, peripheral nervous system diseases, polyneuropathies, polyradiculoneuropathy, radiculopathy, respiratory paralysis, spinal cord diseases, Tourette's syndrome, central nervous system vasculitis, Huntington's disease and stroke.

14. Pharmaceutical composition **characterized in that** it comprises, in a carrier which is pharmaceutically acceptable and compatible with the mode of administration selected for this composition, at least one compound as claimed in any one of claims 1 to 9, which compound can also be in one of its enantiomeric forms or in the form of one of its pharmaceutically acceptable salts.

15. The compound as claimed in claim 1, wherein the compound is:
(S)-N-hydroxy-3-[4-(2-methylquinolin-4-ylmethoxy)benzenesulfonylamino]-2-piperidin-1-yl-propionamide or
(S)-N-hydroxy-3-[4-(2-methylquinolin-4-ylmethoxy)benzenesulfonylamino]-2-morpholin-4-yl-propionamide.
